# EUROPEAN PATENT APPLICATION

(11) **EP 1 270 589 A1**
(43) Date of publication of application: **02.01.2003**
(21) Application number: 01912398.3
(22) Date of filing: 15.03.2001
(51) Int. Cl.: C07K 14/705, C12N 15/12, C12N 1/21, C12N 1/19, C12N 5/10, C12P 21/02, C07K 16/28, C12Q 1/02, G01N 33/50, G01N 33/15

(54) **NOVEL LEUKOTRIENE B4 RECEPTOR**

(30) Priority: 21.03.2000 JP 2000078992; 22.06.2000 JP 2000187978
(71) Applicant: YAMANOUCHI PHARMACEUTICAL CO., LTD., Tokyo 103 (JP)
(72) Inventor: KAMOHARA, Masazumi, Yamanouchi Pharm. Co., Ltd, Tsukuba-shi, Ibaraki 305-8585 (JP); MATSUMOTO, Mitsuyuki, Tsukuba-shi, Ibaraki 305-8585 (JP); TAKASAKI, Jun, Tsukuba-shi, Ibaraki 305-8585 (JP); SAITO, Tetsu, Tsukuba-shi, Ibaraki 305-8585 (JP); OHISHI, Takahide, Tsukuba-shi, Ibaraki 305-8585 (JP)
(74) Representative: HOFFMANN - EITLE
(86) International application number: JP0102060
(87) International publication number: WO01070815

(57) **Abstract**

This invention provides a nucleic acid encoding a novel leukotriene B₄ receptor, a vector containing this nucleic acid, a method for screening a substance capable of modifying the activity of the receptor by using a host cell containing the vector and the receptor, and pharmaceutical compositions for inflammatory diseases containing as the active ingredient a substance capable of modifying the activity of the receptor.

SEQUENCE LISTING

<110> Yamanouchi Pharmaceutical Co.,Ltd.

<120> Novel Leukotrien B4 Receptor

<130> Y0105-PCT

<140>
<141>

<150> JP 2000-078992
<151> 2000-03-21

<150> JP 2000-187978
<151> 2000-06-22

<160> 17

<170> PatentIn Ver. 2.1

<210> 1
<211> 1077
<212> DNA
<213> Homo sapiens

<220>
<221> CDS
<222> (1)..(1077)

<400> 1
atg tcg gtc tgc tac cgt ccc cca ggg aac gag aca ctg ctg agc tgg 48
Met Ser Val Cys Tyr Arg Pro Pro Gly Asn Glu Thr Leu Leu Ser Trp
1               5                   10                  15

aag act tcg cgg gcc aca ggc aca gcc ttc ctg ctg ctg gcg gcg ctg 96
Lys Thr Ser Arg Ala Thr Gly Thr Ala Phe Leu Leu Leu Ala Ala Leu
            20                  25                  30

ctg ggg ctg cct ggc aac ggc ttc gtg gtg tgg agc ttg gcg ggc tgg 144
Leu Gly Leu Pro Gly Asn Gly Phe Val Val Trp Ser Leu Ala Gly Trp
        35                  40                  45

cgg cct gca cgg ggg cga ccg ctg gcg gcc acg ctt gtg ctg cac ctg 192
Arg Pro Ala Arg Gly Arg Pro Leu Ala Ala Thr Leu Val Leu His Leu

50                  55                  60

gcg ctg gcc gac ggc gcg gtg ctg ctg ctc acg ccg ctc ttt gtg gcc    240
Ala Leu Ala Asp Gly Ala Val Leu Leu Leu Thr Pro Leu Phe Val Ala
65                  70                  75                  80

ttc ctg acc cgg cag gcc tgg ccg ctg ggc cag gcg ggc tgc aag gcg    288
Phe Leu Thr Arg Gln Ala Trp Pro Leu Gly Gln Ala Gly Cys Lys Ala
                85                  90                  95

gtg tac tac gtg tgc gcg ctc agc atg tac gcc agc gtg ctg ctc acc    336
Val Tyr Tyr Val Cys Ala Leu Ser Met Tyr Ala Ser Val Leu Leu Thr
            100                 105                 110

ggc ctg ctc agc ctg cag cgc tgc ctc gca gtc acc cgc ccc ttc ctg    384
Gly Leu Leu Ser Leu Gln Arg Cys Leu Ala Val Thr Arg Pro Phe Leu
        115                 120                 125

gcg cct cgg ctg cgc agc ccg gcc ctg gcc cgc cgc ctg ctg ctg gcg    432
Ala Pro Arg Leu Arg Ser Pro Ala Leu Ala Arg Arg Leu Leu Leu Ala
    130                 135                 140

gtc tgg ctg gcc gcc ctg ttg ctc gcc gtc ccg gcc gcc gtc tac cgc    480
Val Trp Leu Ala Ala Leu Leu Leu Ala Val Pro Ala Ala Val Tyr Arg
145                 150                 155                 160

cac ctg tgg agg gac cgc gta tgc cag ctg tgc cac ccg tcg ccg gtc    528
His Leu Trp Arg Asp Arg Val Cys Gln Leu Cys His Pro Ser Pro Val
                165                 170                 175

cac gcc gcc gcc cac ctg agc ctg gag act ctg acc gct ttc gtg ctt    576
His Ala Ala Ala His Leu Ser Leu Glu Thr Leu Thr Ala Phe Val Leu
            180                 185                 190

cct ttc ggg ctg atg ctc ggc tgc tac agc gtg acg ctg gca cgg ctg    624
Pro Phe Gly Leu Met Leu Gly Cys Tyr Ser Val Thr Leu Ala Arg Leu
        195                 200                 205

cgg ggc gcc cgc tgg ggc tcc ggg cgg cac ggg gcg cgg gtg ggc cgg    672
Arg Gly Ala Arg Trp Gly Ser Gly Arg His Gly Ala Arg Val Gly Arg
    210                 215                 220

ctg gtg agc gcc atc gtg ctt gcc ttc ggc ttg ctc tgg gcc ccc tac    720
Leu Val Ser Ala Ile Val Leu Ala Phe Gly Leu Leu Trp Ala Pro Tyr
225                 230                 235                 240

cac gca gtc aac ctt ctg cag gcg gtc gca gcg ctg gct cca ccg gaa 768
His Ala Val Asn Leu Leu Gln Ala Val Ala Ala Leu Ala Pro Pro Glu
                245                 250                 255

ggg gcc ttg gcg aag ctg ggc gga gcc ggc cag gcg gcg cga gcg gga 816
Gly Ala Leu Ala Lys Leu Gly Gly Ala Gly Gln Ala Ala Arg Ala Gly
            260                 265                 270

act acg gcc ttg gcc ttc ttc agt tct agc gtc aac ccg gtg ctc tac 864
Thr Thr Ala Leu Ala Phe Phe Ser Ser Ser Val Asn Pro Val Leu Tyr
        275                 280                 285

gtc ttc acc gct gga gat ctg ctg ccc cgg gca ggt ccc cgt ttc ctc 912
Val Phe Thr Ala Gly Asp Leu Leu Pro Arg Ala Gly Pro Arg Phe Leu
    290                 295                 300

acg cgg ctc ttc gaa ggc tct ggg gag gcc cga ggg ggc ggc cgc tct 960
Thr Arg Leu Phe Glu Gly Ser Gly Glu Ala Arg Gly Gly Gly Arg Ser
305                 310                 315                 320

agg gaa ggg acc atg gag ctc cga act acc cct cag ctg aaa gtg gtg 1008
Arg Glu Gly Thr Met Glu Leu Arg Thr Thr Pro Gln Leu Lys Val Val
                325                 330                 335

ggg cag ggc cgc ggc aat gga gac ccg ggg ggt ggg atg gag aag gac 1056
Gly Gln Gly Arg Gly Asn Gly Asp Pro Gly Gly Gly Met Glu Lys Asp
            340                 345                 350

ggt ccg gaa tgg gac ctt tga 1077
Gly Pro Glu Trp Asp Leu
        355

<210> 2
<211> 358
<212> PRT
<213> Homo sapiens

<400> 2
Met Ser Val Cys Tyr Arg Pro Pro Gly Asn Glu Thr Leu Leu Ser Trp
1               5                   10                  15
Lys Thr Ser Arg Ala Thr Gly Thr Ala Phe Leu Leu Leu Ala Ala Leu
            20                  25                  30
Leu Gly Leu Pro Gly Asn Gly Phe Val Val Trp Ser Leu Ala Gly Trp

35                  40                  45
Arg Pro Ala Arg Gly Arg Pro Leu Ala Ala Thr Leu Val Leu His Leu
    50                  55                  60
Ala Leu Ala Asp Gly Ala Val Leu Leu Leu Thr Pro Leu Phe Val Ala
65                  70                  75                  80
Phe Leu Thr Arg Gln Ala Trp Pro Leu Gly Gln Ala Gly Cys Lys Ala
                85                  90                  95
Val Tyr Tyr Val Cys Ala Leu Ser Met Tyr Ala Ser Val Leu Leu Thr
            100                 105                 110
Gly Leu Leu Ser Leu Gln Arg Cys Leu Ala Val Thr Arg Pro Phe Leu
        115                 120                 125
Ala Pro Arg Leu Arg Ser Pro Ala Leu Ala Arg Arg Leu Leu Leu Ala
    130                 135                 140
Val Trp Leu Ala Ala Leu Leu Leu Ala Val Pro Ala Ala Val Tyr Arg
145                 150                 155                 160
His Leu Trp Arg Asp Arg Val Cys Gln Leu Cys His Pro Ser Pro Val
                165                 170                 175
His Ala Ala Ala His Leu Ser Leu Glu Thr Leu Thr Ala Phe Val Leu
            180                 185                 190
Pro Phe Gly Leu Met Leu Gly Cys Tyr Ser Val Thr Leu Ala Arg Leu
        195                 200                 205
Arg Gly Ala Arg Trp Gly Ser Gly Arg His Gly Ala Arg Val Gly Arg
    210                 215                 220
Leu Val Ser Ala Ile Val Leu Ala Phe Gly Leu Leu Trp Ala Pro Tyr
225                 230                 235                 240
His Ala Val Asn Leu Leu Gln Ala Val Ala Ala Leu Ala Pro Pro Glu
                245                 250                 255
Gly Ala Leu Ala Lys Leu Gly Gly Ala Gly Gln Ala Ala Arg Ala Gly
            260                 265                 270
Thr Thr Ala Leu Ala Phe Phe Ser Ser Ser Val Asn Pro Val Leu Tyr
        275                 280                 285
Val Phe Thr Ala Gly Asp Leu Leu Pro Arg Ala Gly Pro Arg Phe Leu
    290                 295                 300
Thr Arg Leu Phe Glu Gly Ser Gly Glu Ala Arg Gly Gly Gly Arg Ser
305                 310                 315                 320
Arg Glu Gly Thr Met Glu Leu Arg Thr Thr Pro Gln Leu Lys Val Val
                325                 330                 335
Gly Gln Gly Arg Gly Asn Gly Asp Pro Gly Gly Gly Met Glu Lys Asp
            340                 345                 350
Gly Pro Glu Trp Asp Leu
        355

<210> 3

<211> 39
<212> DNA
<213> Artificial Sequence

<220>
<223> Description of Artificial Sequence:an artificially
      synthesized primer sequence

<400> 3
ctagtctaga atgtcggtct gctaccgtcc cccagggaa                                39


<210> 4
<211> 40
<212> DNA
<213> Artificial Sequence

<220>
<223> Description of Artificial Sequence:an artificially
      synthesized primer sequence

<400> 4
ctagtctaga ttatcaaagg tcccattccg gaccgtcctt                               40


<210> 5
<211> 36
<212> DNA
<213> Artificial Sequence

<220>
<223> Description of Artificial Sequence:an artificially
      synthesized primer sequence

<400> 5
atggactaca aggacgacga tgacaagggg atcctg                                   36


<210> 6
<211> 12
<212> PRT
<213> Artificial Sequence

<220>
<223> Description of Artificial Sequence:a FLAG tag amino

acid sequence

<400> 6
Met Asp Tyr Lys Asp Asp Asp Asp Lys Gly Ile Leu
1               5                   10

<210> 7
<211> 22
<212> PRT
<213> Artificial Sequence

<220>
<223> Description of Artificial Sequence:an artificially
      synthesized antigen sequence

<400> 7
Cys Gln Gly Arg Gly Asn Gly Asp Pro Gly Gly Gly Met Glu Lys Asp
1               5                   10                  15

Gly Pro Glu Trp Asp Leu
            20

<210> 8
<211> 29
<212> DNA
<213> Homo sapiens

<400> 8
tgggccaggc gggctgcaag gcggtgtac                                    29

<210> 9
<211> 26
<212> DNA
<213> Homo sapiens

<400> 9
agcgtcacgc tgtagcagcc gagcat                                       26

<210> 10
<211> 29
<212> DNA

<213> Homo sapiens

<400> 10
tgggccaggc gggctgcaag gcggtgtac                                    29


<210> 11
<211> 26
<212> DNA
<213> Homo sapiens

<400> 11
agcgtcacgc tgtagcagcc gagcat                                       26


<210> 12
<211> 20
<212> DNA
<213> Rattus norvegicus

<400> 12
   ccagccagac ccccagcagc                                               20


<210> 13
<211> 21
<212> DNA
<213> Rattus norvegicus

<400> 13
cagcctgcag cgctgtctag c                                            21


<210> 14
<211> 30
<212> DNA
<213> Artificial Sequence

<220>
<223> Description of Artificial Sequence:an artificially
      synthesized primer sequence

<400> 14
ttttactagt atgtctgtct gctaccgtcc                                   30

<210> 15
<211> 32
<212> DNA
<213> Artificial Sequence

<220>
<223> Description of Artificial Sequence:an artificially
      synthesized primer sequence

<400> 15
ttttactagt ctaccattcc tgactgtctt tc                                32


<210> 16
<211> 1077
<212> DNA
<213> Rattus norvegicus

<220>
<221> CDS
<222> (1)..(1077)

<400> 16
atg tct gtc tgc tac cgt ccg cct ggg aat gag acg ctg ctg agt tgg    48
Met Ser Val Cys Tyr Arg Pro Pro Gly Asn Glu Thr Leu Leu Ser Trp
  1               5                  10                  15

aag ggc tcg cgg gcc acc ggc act gcc ttt ctc ctg ctg gcg gcg ttg    96
Lys Gly Ser Arg Ala Thr Gly Thr Ala Phe Leu Leu Leu Ala Ala Leu
             20                  25                  30

ctg gga ctg cca ggc aat ggc ttc gta gtg tgg agc ttg gcg ggc tgg    144
Leu Gly Leu Pro Gly Asn Gly Phe Val Val Trp Ser Leu Ala Gly Trp
         35                  40                  45

cgg ccc acc gcc ggg cgg cca cta gca gcc aca ctt gtg ctg cat ctg    192
Arg Pro Thr Ala Gly Arg Pro Leu Ala Ala Thr Leu Val Leu His Leu
     50                  55                  60

gcg cta gcc gac ggc gcg gtg ctg ctg ctc acg ccg ctc ttt gtg gcc    240
Ala Leu Ala Asp Gly Ala Val Leu Leu Leu Thr Pro Leu Phe Val Ala
 65                  70                  75                  80

ttc ctg agc cga cag gct tgg ccc ctg ggc cag gtg ggc tgc aag gca    288

Phe Leu Ser Arg Gln Ala Trp Pro Leu Gly Gln Val Gly Cys Lys Ala
                85                  90                  95

gtg tac tac gtg tgc gcg ctc agc atg tac gcc agc gtg ctg ctc acc 336
Val Tyr Tyr Val Cys Ala Leu Ser Met Tyr Ala Ser Val Leu Leu Thr
            100                 105                 110

ggc ctg ctc agc ctg cag cgc tgt cta gcg gtc act cgg cct ttc ctg 384
Gly Leu Leu Ser Leu Gln Arg Cys Leu Ala Val Thr Arg Pro Phe Leu
        115                 120                 125

gct ccc cga ctt cgc agc ccg gcc ctg gcc cgc cgc ctg ctg ctg ggg 432
Ala Pro Arg Leu Arg Ser Pro Ala Leu Ala Arg Arg Leu Leu Leu Gly
    130                 135                 140

gtc tgg ctg gcc gcc ctg gtg ctc gcc gtc ccg gcc gcg gtc tac cgc 480
Val Trp Leu Ala Ala Leu Val Leu Ala Val Pro Ala Ala Val Tyr Arg
145                 150                 155                 160

cac ctc tgg ggt gat cgc gtg tgt caa ttg tgc cac cca tcg gcc gtg 528
His Leu Trp Gly Asp Arg Val Cys Gln Leu Cys His Pro Ser Ala Val
                165                 170                 175

cac gct gca gct cat ctg agc ctg gag acc ctg act gcc ttc gtc ctg 576
His Ala Ala Ala His Leu Ser Leu Glu Thr Leu Thr Ala Phe Val Leu
            180                 185                 190

cct ttt ggg acc gtg ctc ggc tgc tac ggc gtg acg ctg gcg cgg ttg 624
Pro Phe Gly Thr Val Leu Gly Cys Tyr Gly Val Thr Leu Ala Arg Leu
        195                 200                 205

cgg gga gcg cgc tgg ggc tcg ggg cga caa ggc acg cgg gtg ggt cgt 672
Arg Gly Ala Arg Trp Gly Ser Gly Arg Gln Gly Thr Arg Val Gly Arg
    210                 215                 220

ctg gtg agc gcc atc gta ctg gcc ttt ggc ttg ctc tgg gcc ccc tac 720
Leu Val Ser Ala Ile Val Leu Ala Phe Gly Leu Leu Trp Ala Pro Tyr
225                 230                 235                 240

cac gcg gtc aat ctc cta cag gcg gtg gcc gcg ctc gct ccg ccg gaa 768
His Ala Val Asn Leu Leu Gln Ala Val Ala Ala Leu Ala Pro Pro Glu
                245                 250                 255

gga ccc cta gcc agg ctc ggt ggg gcg ggc cag gca gcg cgc gct gga 816
Gly Pro Leu Ala Arg Leu Gly Gly Ala Gly Gln Ala Ala Arg Ala Gly

            260                 265                 270

act aca gcc ttg gct ttc ttt agt tcc agc gtc aac ccg gtg ctc tac 864
Thr Thr Ala Leu Ala Phe Phe Ser Ser Ser Val Asn Pro Val Leu Tyr
        275                 280                 285

gtc ttt act gcg ggt gat ttg ctg ccg cgg gcg ggg cct cgg ttc ctc 912
Val Phe Thr Ala Gly Asp Leu Leu Pro Arg Ala Gly Pro Arg Phe Leu
    290                 295                 300

act cga ctc ttc gaa ggc tct ggg gag gcc cga gta ggc agc cgc tct 960
Thr Arg Leu Phe Glu Gly Ser Gly Glu Ala Arg Val Gly Ser Arg Ser
305                 310                 315                 320

agg gag ggt acc atg gag ctc cga act acc ccc agg ctg aaa gta gtg 1008
Arg Glu Gly Thr Met Glu Leu Arg Thr Thr Pro Arg Leu Lys Val Val
                325                 330                 335

ggt cag ggc agg ggc tat gga gac cct gga ggt ggg ggc agg atg gag 1056
Gly Gln Gly Arg Gly Tyr Gly Asp Pro Gly Gly Gly Gly Arg Met Glu
            340                 345                 350

aaa gac agt cag gaa tgg tag 1077
Lys Asp Ser Gln Glu Trp
        355

<210> 17
<211> 358
<212> PRT
<213> Rattus norvegicus

<400> 17
Met Ser Val Cys Tyr Arg Pro Pro Gly Asn Glu Thr Leu Leu Ser Trp
1               5                   10                  15
Lys Gly Ser Arg Ala Thr Gly Thr Ala Phe Leu Leu Leu Ala Ala Leu
            20                  25                  30
Leu Gly Leu Pro Gly Asn Gly Phe Val Val Trp Ser Leu Ala Gly Trp
        35                  40                  45
Arg Pro Thr Ala Gly Arg Pro Leu Ala Ala Thr Leu Val Leu His Leu
    50                  55                  60
Ala Leu Ala Asp Gly Ala Val Leu Leu Leu Thr Pro Leu Phe Val Ala
65                  70                  75                  80
Phe Leu Ser Arg Gln Ala Trp Pro Leu Gly Gln Val Gly Cys Lys Ala
                85                  90                  95

Val Tyr Tyr Val Cys Ala Leu Ser Met Tyr Ala Ser Val Leu Leu Thr
            100                 105                 110
Gly Leu Leu Ser Leu Gln Arg Cys Leu Ala Val Thr Arg Pro Phe Leu
        115                 120                 125
Ala Pro Arg Leu Arg Ser Pro Ala Leu Ala Arg Arg Leu Leu Leu Gly
    130                 135                 140
Val Trp Leu Ala Ala Leu Val Leu Ala Val Pro Ala Ala Val Tyr Arg
145                 150                 155                 160
His Leu Trp Gly Asp Arg Val Cys Gln Leu Cys His Pro Ser Ala Val
                165                 170                 175
His Ala Ala Ala His Leu Ser Leu Glu Thr Leu Thr Ala Phe Val Leu
            180                 185                 190
Pro Phe Gly Thr Val Leu Gly Cys Tyr Gly Val Thr Leu Ala Arg Leu
        195                 200                 205
Arg Gly Ala Arg Trp Gly Ser Gly Arg Gln Gly Thr Arg Val Gly Arg
    210                 215                 220
Leu Val Ser Ala Ile Val Leu Ala Phe Gly Leu Leu Trp Ala Pro Tyr
225                 230                 235                 240
His Ala Val Asn Leu Leu Gln Ala Val Ala Ala Leu Ala Pro Pro Glu
                245                 250                 255
Gly Pro Leu Ala Arg Leu Gly Gly Ala Gly Gln Ala Ala Arg Ala Gly
            260                 265                 270
Thr Thr Ala Leu Ala Phe Phe Ser Ser Ser Val Asn Pro Val Leu Tyr
        275                 280                 285
Val Phe Thr Ala Gly Asp Leu Leu Pro Arg Ala Gly Pro Arg Phe Leu
    290                 295                 300
Thr Arg Leu Phe Glu Gly Ser Gly Glu Ala Arg Val Gly Ser Arg Ser
305                 310                 315                 320
Arg Glu Gly Thr Met Glu Leu Arg Thr Thr Pro Arg Leu Lys Val Val
                325                 330                 335
Gly Gln Gly Arg Gly Tyr Gly Asp Pro Gly Gly Gly Gly Arg Met Glu
            340                 345                 350
Lys Asp Ser Gln Glu Trp
        355

## Description

### Technical Field

This invention relates to a novel leukotriene B₄ receptor, a nucleic acid encoding the receptor, a vector containing the nucleic acid, a method for screening a substance capable of modifying the activity of the receptor by using a host cell containing the vector and the receptor, and pharmaceutical compositions for inflammatory diseases containing as the active ingredient a substance capable of modifying the activity of the receptor.

### Background of the Invention

It is known that leukotriene B₄ (LTB₄) is a strong leukocyte-activating factor and taking important roles in inflammatory immune reaction, infection protection and the like (Chen, X.S. *et al.* (1994) *Nature*, 372, pp. 179 - 182), and it is known also that it causes leukocytes to release lysosomal enzymes, produce active oxygen and adhere to vascular endothelial cells (Palmblad, J. *et al.* (1994) *J. Immunol*., 152, pp. 262 - 269). LTB₄ is produced mainly in myelocyte system cells such as leukocytes, but it is produced also in parenchymal cells such as of the intestinal tract and the lungs when leukotriene A₄ (LTA₄) as the LTB₄ precursor is supplied. Based on such actions, it is considered that LTB₄ is concerned in the onset, advance and exacerbation of rheumatoid arthritis, edema, glomerulonephritis and the like renal diseases, bronchitis, airway oversensitivity and the like respiratory diseases, eczema, dermatitis and the like skin diseases, psoriasis, inflammatory bowel disease, ulcerative colitis and the like intestinal diseases, cerebral myelitis and the like central diseases (Koza Prostaglandin 3 (1988), 225 - 227, 484 - 486, edited by N. Katori, M. Murota and S. Yamamoto; William T.J. (1999) Novel Inhibitors of Leukotrienes, 299 - 316). Actuary, a large number of LTB₄ receptor antagonists (Negro, J.M. *et al*. (1997) *Allergol. Immunopathol. Mdr.,* 25, 104 - 112; Kishikawa, K. *et al.* (1995) *Adv. Prostaglandin Thromboxane Leukot. Res*., 23, 279 - 281) have been studied and developed with the aim of obtaining antiinflammatory drugs. As receptors which specifically recognize LTB₄, human BLT (Yokomizo, T. *et al*. (1997) Nature, 387, 620 - 624) and mouse BLT (Martin, V. *et al.* (1999) *J. Biol. Chem*., 274, 8597 - 8603) have so far been isolated and identified, but the presence of other LTB₄ receptors is not known.

### Disclosure of the Invention

The invention aims at providing a novel leukotriene B₄ (LTB₄) receptor and a nucleic acid encoding the receptor. The invention also aims at providing a method for screening a substance capable of modifying the activity of the receptor by using the novel LTB₄ receptor and pharmaceuticals containing as the active ingredient a substance capable of modifying the activity of the receptor, particularly pharmaceuticals for inflammatory diseases.

As a result of intensive studies carried out for resolving the above problems, the present inventors have succeeded in isolating nucleic acids coding for novel human and rat LTB₄ receptors which are different from already known LTB₄ receptors and have determined their nucleotide sequences and deduced amino acid sequences. Next, vectors containing nucleic acids coding for the receptors and host cells containing the vectors were prepared in succession, production of novel recombinant LTB₄ receptors was made possible by expressing LTB₄ receptors using the host cells, and then it was confirmed that the receptors induce inflammation based on the finding that the receptors have cell migration activity. Also, a method for producing antibodies for the receptors was established and the antibodies were obtained. In addition, a method for screening substances capable of modifying the activity of the receptors was established, substances having antagonist activity for the receptors were obtained by the screening method and then it was confirmed that these substances are effective in preventing and/or treating inflammatory diseases, thus accomplishing the invention.

Accordingly, the invention relates to:
[1] A leukotriene B₄ receptor, which is represented by the amino acid sequence described in SEQ ID NO:2, or the amino acid sequence described in SEQ ID NO:2 in which one or more amino acid(s) residues are substituted, deleted and/or inserted at one or more position(s) and showing the same activity of the leukotriene B₄ receptor represented by the amino acid sequence described in SEQ ID NO:2,
[2] the leukotriene B₄ receptor described in [1], which is represented by the amino acid sequence described in SEQ ID NO:2,
[3] a leukotriene B₄ receptor, which is a protein encoded by a DNA that hybridizes with the DNA represented by the nucleotide sequence described in SEQ ID NO:1 or SEQ ID NO:16 under a stringent condition and showing the same activity of the leukotriene B₄ receptor represented by the amino acid sequence described in SEQ ID NO:2,
[4] the leukotriene B₄ receptor described in [3], which is a protein represented by the amino acid sequence described in SEQ ID NO:2,
[5] the leukotriene B₄ receptor described in [3], which is a protein represented by the amino acid sequence described in SEQ ID NO:17,
[6] a nucleic acid which encodes the amino acid sequence of the leukotriene B₄ receptor described in [1] or [3],
[7] a vector which contains the nucleic acid described in [6],
[8] a host cell which contains the vector described in [7],
[9] a method for producing the leukotriene B₄ receptor described in [1] or [3], characterized in that it uses the host cell described in [8],
[10] an antibody which binds to the leukotriene B₄ receptor described in [1] or [3],
[11] a method for screening a substance capable of modifying the activity of the leukotriene B₄ receptor described in [1] or [3], characterized in that the leukotriene B₄ receptor is allowed to contact with an agent to be tested,
[12] the screening method described in [11], which comprises a step for contacting the leukotriene B₄ receptor described in [1] or [3] with an agent to be tested in the presence of a ligand and a step for measuring changes in the receptor activity,
[13] the screening method described in [11], which comprises a step for contacting the leukotriene B₄ receptor described in [1] or [3] with an agent to be tested in the presence of a ligand and a step for measuring binding inhibition activity of the ligand for the receptor,
[14] the screening method described in [11], wherein the substance capable of modifying the activity of the leukotriene B₄ receptor described in [1] or [3] is a substance for preventing and/or treating an inflammatory disease,
[15] an antagonist of the leukotriene B₄ receptor described in [1] or [3], which is capable of being selected by the method described in [11],
[16] a pharmaceutical composition for an inflammatory disease (excluding a pharmaceutical composition for an inflammatory disease in which 4-octyloxybenzenecarboximidoamide hydrochloride is the active ingredient), which contains as the active ingredient a substance that is capable of modifying the activity of the leukotriene B₄ receptor described in [1] or [3] and is capable of being selected by the method described in [11], and
[17] the pharmaceutical composition for an inflammatory disease described in [16] (excluding a pharmaceutical composition for an inflammatory disease in which 4-octyloxybenzenecarboximidoamide hydrochloride is the active ingredient), wherein the substance capable of modifying the activity is a substance having antagonist activity.

As illustrative examples of the inflammatory disease, bronchitis, dermatitis, psoriasis, ulcerative colitis, rheumatoid arthritis and edema can be cited.

The LTB₄ receptor of the invention can be used for the screening of a substance which modifies the activity of the receptor. Among substances which modify the receptor, a substance having antagonist activity for the receptor, particularly a substance which inhibits cell migration activity, is useful for the prevention and/or treatment of inflammatory diseases (bronchitis, dermatitis, psoriasis, ulcerative colitis, rheumatoid arthritis and edema).

According to a BLAST (basic local alignment search tool) (S.F. Altschul *et al.,* (1990) *J. Mol. Biol.,* 215, 403 - 410) retrieving result of GENBANK and SwissProt, the nucleic acid of the invention represented by the nucleotide sequence (1,077 base pairs) described in SEQ ID NO:16 and the protein represented by the amino acid sequence (358 amino acids) described in SEQ ID NO:17 are novel. Also, the nucleotide sequence (1,077 base pairs) of the invention described in SEQ ID NO:1 and the amino acid sequence (358 amino acids) described in SEQ ID NO:2 were disclosed in international publications WO 00/22131 and WO 00/31258 which had been published after the priority date of this application, but the specifications merely describe the nucleotide sequence and a deduced amino acid sequence encoded thereby and do not describe about an illustrative and specific use of the protein represented by the amino acid sequence described in SEQ ID NO:2, the fact that a novel LTB₄ receptor is encoded by the nucleotide sequence described in SEQ ID NO:1, an illustrative production method of the LTB₄ receptor represented by the amino acid sequence and obtainment of the LTB₄ receptor represented by the amino acid sequence.

The "LTB₄ receptor" as used in this specification represents "LTB₄ receptor protein".

The LTB₄ receptor of the invention includes,
(1) an LTB₄ receptor represented by the amino acid sequence described in SEQ ID NO:2,
(2) an LTB₄ receptor represented by the amino acid sequence described in SEQ ID NO:17,
(3) an LTB₄ receptor which has the amino acid sequence described in SEQ ID NO:2 in which one or more of amino acid residue(s) are substituted, deleted and/or inserted at one or more of position(s) and which also "shows the same activity of the LTB₄ receptor represented by the amino acid sequence described in SEQ ID NO:2" (hereinafter to be referred to as an "equivalent" of the protein represented by the amino acid sequence described in SEQ ID NO:2),
(4) an LTB₄ receptor which is a protein encoded by a DNA that hybridizes with the DNA represented by the nucleotide sequence described in SEQ ID NO:1 or SEQ ID NO:16 "under a stringent condition" and which also "shows the same activity of the LTB₄ receptor represented by the amino acid sequence described in SEQ ID NO:2" (hereinafter to be referred to as a "hybridizing equivalent" of the protein represented by the amino acid sequence described in SEQ ID NO:2), and
(5) an LTB₄ receptor which is represented by an amino acid sequence having 92% or more of homology with the amino acid sequence described in SEQ ID NO:2 or SEQ ID NO:17 and which also "shows the same activity of the LTB₄ receptor represented by the amino acid sequence described in SEQ ID NO:2" (hereinafter to be referred to as a "homologous protein" of the protein represented by the amino acid sequence described in SEQ ID NO:2).

The term "shows the same activity of the LTB₄ receptor represented by the amino acid sequence described in SEQ ID NO:2" means that production of cAMP by forskolin stimulation is inhibited in a dose-dependent manner in the presence of LTB₄ by the method described in Example 5, and the response of LTB₄ under the conditions described in Example 5 is preferably EC₅₀ = 50 nM or less, more preferably EC₅₀ = 15 nM or less. In addition, regarding the term "shows the same activity of the LTB₄ receptor represented by the amino acid sequence described in SEQ ID NO:2", it is more desirable that it specifically binds to [³H]LTB₄ by the method described in Example 8.

The "equivalent" of the protein represented by the amino acid sequence described in SEQ ID NO:2 is an LTB₄ receptor which is represented by an amino acid sequence in which the amino acid sequence described in SEQ ID NO:2 has substitution, deletion or insertion of amino acids with preferably from 1 to 10, more preferably from 1 to 7, most preferably from 1 to 5, of amino acids and which also "shows the same activity of the LTB₄ receptor represented by the amino acid sequence described in SEQ ID NO:2".

Regarding the "stringent condition" under which a DNA coding for a "hybridizing equivalent" of the protein represented by the amino acid sequence described in SEQ ID NO:2 hybridizes with the DNA represented by the nucleotide sequence described in SEQ ID NO:1 or SEQ ID NO: 16, it is generally a hybridization condition of "5 x SSC, 5 x Denhaldts, 0.5% SDS, 40% formamide, 42°C" and a washing condition of "0.5 x SSC, 0.1% SDS, 55°C", and more stringent condition is a washing condition of "0.1 x SSC, 0.1% SDS, 65°C".

Homology of the amino acid sequence of an "homologous protein" of the protein represented by the amino acid sequence described in SEQ ID NO:2 with the amino acid sequence described in SEQ ID NO:2 or SEQ ID NO:17 is 92% or more, but is preferably 95% or more and more preferably 97% or more. In this connection, the homology was calculated using the Clustal method (Higgins D.G. and Sharo P.M. (1989) *Comput. Appl. Biosci*., 5, 151 - 3) of MegAlign ver 4.00 (mfd. by DNASTAR) in accordance with the default parameters. Illustratively, it was calculated under conditions of a Gap penalty value of 10 and a Gap length penalty value of 10 as the multiple alignment parameters and a K tuple value of 1, a Gap penalty value of 3, a Window value of 5 and a Diagonal Saved value of 5 as the pair wise alignment parameters.

Origin of the LTB₄ receptor of the invention is not limited to human and rat. For example, an LTB₄ receptor originated from an organism other than human and rat (e.g., mouse, hamster or dog) and a receptor artificially modified by genetic engineering techniques based on the sequence described in SEQ ID NO:2 or SEQ ID NO:17 are also included in the LTB₄ receptor of the invention, with the proviso that they correspond to any one of the LTB₄ receptors of the invention of the aforementioned items (1) to (5).

Also, it is desirable that the LTB₄ receptor of the invention is a recombinant LTB₄ receptor.

In addition, the nucleic acid of the invention is not particularly limited, with the proviso that it is a nucleic acid represented by the nucleotide sequence coding for any one of the LTB₄ receptors of the invention of the aforementioned items (1) to (5).

The "nucleic acid" includes DNA and RNA, but DNA is desirable.

### 1) Methods for producing nucleic acid coding for the LTB₄ receptor of the invention

The nucleic acid of the invention can be produced by the following methods.

### a) First production method

mRNA is extracted from human cells or rat cells, or human or rat tissues, having the ability to produce the LTB₄ receptor of the invention. Next, using this mRNA as the template, two primers interposing the LTB₄ receptor mBNA or a partial mRNA region are prepared. By carrying out reverse transcriptase-polymerase chain reaction (to be referred to as RT-PCR hereinafter), the receptor cDNA or a part thereof can be obtained. Thereafter, the receptor can be produced by integrating the thus obtained human or rat LTB₄ receptor cDNA or a part thereof into an appropriate expression vector and expressing it in a host cell.

Firstly, from a cell or tissue such as human spleen having the ability to produce the LTB₄ receptor of the invention, mRNA molecules including the one coding for the receptor are extracted by a known method. As the extraction method, guanidine thiocyanate hot phenol method, guanidine thiocyanate-guanidine hydrochloride method and the like can be exemplified, and preferably, guanidine thiocyanate cesium chloride method can be cited. The cell or tissue having the ability to produce the receptor can be specified by methods such as northern blot technique which uses a nucleic acid having a nucleotide sequence encoding the receptor, or a part thereof, and western blotting that uses an antibody specific for the receptor.

Purification of mBNA can be carried out in accordance with a usual method; for example, mRNA can be purified by adsorbing and eluting it using an oligo(dT)-cellulose column. Also, the mRNA can be further fractionated by a sucrose density gradient centrifugation or the like method. Alternatively, a commercially available already extracted mBNA preparation may be used without extracting the mRNA.

Next, a single-stranded cDNA is synthesized by carrying out reverse transcriptase reaction of the thus purified mRNA in the presence of a random primer or oligo(dT) primer. This synthesis can be carried out by a conventional method. Using two primers interposing a partial region of the gene of interest, the thus obtained single-stranded cDNA is subjected to polymerase chain reaction (Saiki, R.K. et *al.* (1988) *Science,* 239, 487 - 491; to be referred to as PCR hereinafter) to amplify the LTB₄ receptor DNA of interest. The thus obtained DNA is fractionated by an agarose gel electrophoresis or the like method. As occasion demands, a DNA fragment of interest can also be obtained by digesting the DNA with restriction enzymes and the like and then ligating the digests.

### b) Second production method

In addition to the production method described in a), a nucleic acid coding for the LTB₄ receptor of the invention can also be produced using conventional genetic engineering techniques. Firstly, a single-stranded cDNA is synthesized using the mRNA obtained by the method described in a) as the template and using reverse transcriptase, and then a double-stranded cDNA is synthesized from the single-stranded cDNA. Examples of the method include S1 nuclease method (Efstratiadis, A. *et al.* (1976), *Cell,* 7, 279 - 288), Land method (Land, H. *et al*. (1981) *Nucleic Acids Res.,* 9, 2251 - 2266), O. Joon Yoo method (Yoo, O.J. *et al.* (1983), *Proc. Natl. Acad. Sci. USA,* 79, 1049 - 1053), Okayama-Berg method (Okayama, H. and Berg, P. (1982) *Mol. Cell. Biol.,* 2, 161 - 170) and the like.

Next, a recombinant plasmid obtained by the above method is transformed by introducing it into an *Escherichia coli* strain, e.g., DH5α, and then the transformants can be selected making use of tetracycline resistance or ampicillin resistance as the marker. The transformation of a host cell, when the host cell is *E*. *coli* for example, can be carried out by the Hanahan's method (Hanahan, D. (1983) *J. Mol. Biol.,* 166, 557 - 580), namely a method in which the recombinant DNA is added to competent cells prepared by allowing CaCl₂ and MgCl₂ or RbCl to coexist. In this connection, not only a plasmid but a lambda or the like phage vector can also be used as the vector.

Various methods shown below can be employed as the method for selecting a strain having the LTB₄ receptor DNA of interest from the thus obtained transformants.

### (1) A screening method which uses a synthetic oligonucleotide probe

An oligonucleotide which corresponds to the entire or a partial portion of the LTB₄ receptor of the invention is synthesized (in this case, it may be either a nucleotide sequence derived by the use of codon usage or plurality of nucleotide sequences by combining possible nucleotide sequences), this is used as a probe (labeled with ³²P or ³³P) and allowed to undergo hybridization on a nitrocellulose filter to which DNA samples of the transformants are denatured and fixed, and then the thus obtained positive strains are screened and selected.

### (2) A screening method which uses a probe prepared by polymerase chain reaction

By synthesizing sense primer and antisense primer oligonucleotides which correspond to a part of the LTB₄ receptor of the invention and carrying out PCR using them in combination, a DNA fragment coding for the entire or a partial portion of the LTB₄ receptor of interest is amplified. As the template DNA to be used herein, a cDNA synthesized by reverse transcription reaction from mRNA of a cell which produces the LTB₄ receptor or a genomic DNA can be used. The DNA fragment prepared in this manner is labeled with ³²P or ³³P, and using this as the probe, colony hybridization or plaque hybridization is carried out to select a clone of interest.

### (3) A selection method which uses an antibody for the LTB₄ receptor of the invention

A cDNA is integrated into an expression vector in advance, protein is produced on the surface of transformants, and then a strain of interest is selected by detecting the desired LTB₄ receptor producing strain using an antibody for the LTB₄ receptor of the invention and a secondary antibody for the antibody.

The method for collecting a DNA coding for the LTB₄ receptor of the invention from the thus obtained transformant of interest can be carried out in accordance with a known method (Maniatis, T. *et al*. (1982): "Molecular Cloning - A Laboratory Manual" Cold Spring Harbor Laboratory, NY). For example, it can be carried out by separating a fraction corresponding to a plasmid DNA from cells and cutting out a cDNA region from the plasmid DNA.

### c) Third production method

A nucleic acid represented by a nucleotide sequence coding for the amino acid sequence described in SEQ ID NO:2 or SEQ ID NO:17 can also be produced by ligating DNA fragments produced by a chemical synthesis method. Each DNA fragment can be synthesized using a DNA synthesizer (e.g., Oligo 1000M DNA Synthesizer (Beckman), or 394 DNA/RNA Synthesizer (Applied Biosystems) or the like).

### d) Fourth production method

In order to express functions of the LTB₄ receptor of the invention, it is not always necessary that it has all of the amino acid sequence described in SEQ ID NO:2 or SEQ ID NO:17. It is considered that genes of eucaryote generally show polymorphism as known in interferon gene and the like (*e.g.,* cf. Nishi, T. *et al.* (1985) *J. Biocbem*., 97, 153 - 159), and there are cases in which one or plurality of amino acids are substituted due to this polymorphism. Thus, in the case of receptors in which one or plurality of amino acid residues are substituted, deleted or inserted at one or plurality of positions in the amino acid sequence described in SEQ ID NO:2 or SEQ ID NO:17, it is possible that they show the same activity of the LTB₄ receptor represented by the amino acid sequence described in SEQ ID NO:2. As described in the foregoing, these receptors are called "equivalents" of the protein represented by the amino acid sequence described in SEQ ID NO:2 and included in the invention, and nucleic acids represented by the nucleotide sequences coding for them are also included in the invention. Such nucleic acids of the invention can also be produced by chemical synthesis of the nucleic acids based on the sequence information on the LTB₄ receptor of the invention in accordance, e.g., with the phosphite triester method (Hunkapiller, M. *et al.* (1984) *Nature,* 10, 105 - 111) or the like usual method. In this connection, codons for desired amino acids are by themselves well known and their selection is optional, and they can be determined, e.g., in accordance with a conventional method taking codon usage of a host to be used into consideration (Crantham, R. *et al.* (1981) *Nucleic Acids Res*., 9, r43 - r74). In addition, partial modification of codons of these nucleotide sequences can be carried out in accordance with a conventional method such as site specific mutagenesis (Mark, D.F. *et al.* (1984) *Proc. Natl. Acad. Sci. USA*, 81, 5662 - 5666) which uses a primer comprised of a synthetic oligonucleotide coding for a desired modification.

As another embodiment of the method for producing a nucleic acid represented by a nucleotide sequence coding for an "equivalent" of the protein represented by the amino acid sequence described in SEQ ID NO:2, a method which uses a hybridization technique or a gene amplification technique can be exemplified. That is, it can be generally carried out by those skilled in the art to isolate a DNA having high homology from the sequence described in SEQ ID NO:1 or SEQ ID NO:16 or from a DNA sample derived from the same or different organism species, prepared based on a part thereof, making use of the hybridization technique (Current Protocols in Molecular Biology, edit. Ausubel *et al.* (1987), publish. John Wily & Sons, Section 6.3 - 6.4), thereby obtaining a nucleic acid represented by a nucleotide sequence coding for the "equivalent" and obtaining the "equivalent" using the same. As described in the foregoing, such a LTB₄ receptor, which is a protein encoded by a DNA that hybridizes with the DNA described in SEQ ID NO:1 or SEQ ID NO:16 under a stringent condition and which also shows the same activity of the LTB₄ receptor represented by the amino acid sequence described in SEQ ID NO:2, is called a "hybridizing equivalent" of the protein represented by the amino acid sequence described in SEQ ID NO:2, and a nucleic acid coding for the same is also included in the invention.

As the organism for isolating the nucleic acid of the invention, mouse, rabbit, domestic fowl, swine, bovine and the like can be exemplified in addition to human and rat, though not limited thereto.

Amino acid sequence of the protein encoded by a DNA isolated by making use of the hybridization technique generally has high homology with the amino acid sequence described in SEQ ID NO:2 or SEQ ID NO:17. The high homology means a sequence homology of at least 92% or more, preferably 95% or more and more preferably 97% or more.

In addition, it is possible to isolate a DNA fragment having high homology with the DNA sequence coding for the amino acid sequence described in SEQ ID NO:2 or SEQ ID NO:17, making use of a gene amplification technique (PCR) (Current Protocols in Molecular Biology, edit. Ausubel *et al*. (1987), publish. John Wily & Sons, Section 6.1 - 6.4) by designing primers based on a part of SEQ ID NO:1 or SEQ ID NO:16, and to obtain an LTB₄ receptor showing the same activity of the LTB₄ receptor represented by the amino acid sequence described in SEQ ID NO:2, using the isolated DNA.

Sequence determination of the DNA obtained by the above methods a) to d) can be carried out, e.g., by the Maxam-Gilbert's chemical modification method (Maxam, A.M. and Gilbert, W. (1980): "Methods in Enzymology", 65, 499 - 559) or the dideoxy nucleotide chain termination method (Messing, J. and Vieira, J. (1982) *Gene,* 19, 269 - 276) which uses M13.

### 2) Methods for producing the vector, host cell and LTB₄ receptor of the invention

The vector, host cell and LTB₄ receptor of the invention can be obtained by the following methods.

Transformation of a host cell with a fragment which contains the nucleic acid coding for the LTB₄ receptor of the invention isolated by the method described in 1) can be carried out by again integrating it into an appropriate vector DNA. Also, it is possible to effect expression of the DNA in respective host cells by introducing an appropriate promoter and a sequence concerning gene expression into the vector.

For example, cells of a vertebrate, an insect, a yeast and the like are included in the eucaryote host cells, and as the vertebrate cells, a COS cell as a monkey cell (Gluzman, Y. (1981) *Cell,* 23, 175 - 182), a Chinese hamster ovary (CHO) cell and a dihydrofolate reductase deficient strain of the same cell (CHO-dhfr (-)) (Urlaub, G. and Chasin, L.A. (1980) *Proc. Natl. Acad. Sci. USA*, 77, 4216 - 4220), a human fetal kidney HEK293 cell and a 293-EBNA cell (mfd. by Invitrogen) in which EBNA-1 gene of Epstein Barr Virus is introduced into the same cell, and the like are frequently used, of which the HEK293 cell and CHO cell used in Examples are particularly preferable.

An expression vector which can be used in vertebrate cells generally has a promoter positioned at upstream of the gene to be expressed, an RNA splicing site, a polyadenylation site, a transcription termination sequence and the like, and it may further has a replication origin as occasion demands. Examples of the expression vector include pSV2dhfr having SV40 early promoter (Subramini, S. *et al.* (1981) *Mol. Cell. Biol.,* 1, 854 - 864), pEF-BOS having human elongation factor promoter (Mizushima, S. and Nagata, S. (1990) *Nucleic Acids Res*., 18, 5322), pCEP4 having cytomegalovirus promoter (mfd. by Invitrogen) and the like, of which the pEF-BOS used in Examples is particularly desirable.

When 293-EBNA cell is used as the host cell, desired transformant cells can be obtained by using pCEP4 (Invitrogen) or the like expression vector which has the Epstein Barr Virus replication origin and can perform autonomous growth in the 293-EBNA cell. When CHO cell is used as the host cell, transformant cells capable of stably producing LTB₄ receptor can be obtained by co-transfecting the expression vector together with a vector capable of expressing neo gene which functions as a G418 marker, such as pRSVneo (Sambrook, J. *et al.* (1989): "Molecular Cloning - A Laboratory Manual" Cold Spring Harbor Laboratory, NY), pSV2-neo (Southern, P.J. and Berg, P. (1982) *J. Mol. Appl. Genet*., 1, 327 - 341) or the like, and selecting G418-resistant colonies. In this connection, a cell strain (CHO-dhfr (-)) lacking in dihydrofolate reductase (dhfr) as an enzyme essential for the *de novo* synthesis of nucleic acids was particularly used in the Examples. Illustratively, making use of the fact that the CHO-dhfr (-) strain cannot survive in a nucleic acid-free medium, transformant cells capable of stably producing LTB₄ receptor can be obtained by transfecting an expression vector pEF-BOS-dhfr-JULF2 prepared by integrating dhfr into pEF-BOS, and then using a nucleic acid-free medium as the selection medium. In addition, it is possible to obtain transformant cells capable of stably producing highly expressed LTB₄ receptor, by adding methotrexate which is a competitive inhibitor of dhfr.

The desired transformant obtained in the above can be cultured in accordance with a conventional method, and the LTB₄ receptor of the invention is produced by this culturing in the cell or on the cell surface. As the medium to be used in the culturing, various conventionally used media can be optionally selected in response to the employed host cells, and in the case of 293-EBNA cell for example, a medium prepared by adding G418 to Dulbecco's Modified Eagle's minimum essential medium (DMEM) or the like medium supplemented with fetal bovine serum (FBS) or the like serum component can be used.

The LTB₄ receptor of the invention thus produced in the cell or on the cell surface of a transformant can be isolated and purified therefrom by various known separation techniques making use of physical properties and chemical properties of the receptor. Illustrative examples of the techniques include usual treatment with a protein precipitant, ultrafiltration, various types of liquid chromatography such as molecular sieve chromatography (gel filtration), adsorption chromatography, ion exchanger chromatography, affinity chromatography, high performance liquid chromatography (HPLC) and the like, dialysis, combinations thereof and the like, which are carried out after solubilizing a membrane fraction containing the receptor. In this connection, the membrane fraction can be obtained in accordance with a conventional method. For example, it can be obtained by culturing cells expressing the LTB₄ receptor of the invention on the surface, suspending the resulting cells in a buffer and then homogenizing and centrifuging them. Also, by solubilizing the LTB₄ receptor with a solubilizing agent as mild as possible (CHAPS, Triton X-100, digitonin or the like), characteristics of the receptor can be maintained after the solubilization.

Confirmation of expression, confirmation of intracellular location, purification and the like of the LTB₄ receptor of the invention become possible by expressing the LTB₄ receptor through its in-frame fusion with a marker sequence. Examples of the marker sequence include FLAG epitope, Hexa-Histidine tag, Hemagglutinin tag, myc epitope and the like. Also, when a specific sequence recognizable by enterokinase, factor Xa, thrombin or the like protease is inserted between the marker sequence and LTB₄ receptor, it becomes possible to cut out and remove the marker sequence moiety with these proteases. For example, there is a report stating that muscarine acetylcholine receptor and Hexa-Histidine tag were connected with a thrombin-recognizing sequence (Hayashi, M.K. and Haga, T. (1996) J. Biochem., 120, 1232 - 1238).

### 3) Method for screening substances (compounds, peptides and antibodies) which modify the activity of the LTB₄ receptor of the invention

A method for screening substances (compounds, peptides and antibodies) which modify the activity of the LTB₄ receptor is included in the invention. This screening method comprised of steps in which the LTB₄ receptor (cell or cell membrane expressing the receptor, or a purified sample of the receptor) produce by the above method 2) is used, an agent to be tested is added to a system for measuring an index of the modification of the receptor in response to a physiological characteristic of the receptor, the receptor and drug to be tested are allowed to contact with each other, and the index is measured by a means, e.g., by measuring changes in the activity of the receptor. As the drug to be tested which can be subjected to the screening method of the invention, for example,
various commercially available compounds such as those which were used in Example 7,
various known compounds registered in chemical files, peptides, and compounds obtained by combinatorial chemistry techniques (Terrett, U.K. et al. (1995) Tetrahedron, 51, 8135 - 8137),
random peptides prepared by applying phage display (Felici, F. *et al.* (1991) *J. Mol. Biol*., 222, 301 - 310) and the like methods, culture supernatants of microorganisms, natural components derived from plants and marine organisms, animal tissue extracts, and
compounds or peptides prepared by chemically or biologically modifying a peptide, can be used. The substances which modify the activity of the LTB₄ receptor of the invention are roughly divided into a substance having agonist activity for the receptor, namely a substance which accelerates the activity of the receptor, and a substance having antagonist activity for the receptor, namely a substance which inhibits the activity of the receptor, by measuring changes in the activity of the receptor, and the screening method of the invention can select any one of the substances having the agonist activity and substances having antagonist activity for the receptor. The screening method of the invention is more suited for the selection of substances having antagonist activity for the receptor.

Also preferred is a method in which substances which bind to the LTB₄ receptor of the invention are screened as a first screening and then screened by a second screening by measuring changes in the activity of the receptor.

An illustrative example of the method for screening substances which bind to the LTB₄ receptor of the invention is the method of following a), preferably the method described in Example 8. Also, illustrative examples of the method for measuring changes in the activity of the LTB₄ receptor of the invention include the methods of following b) and c), of which preferred is the screening method of c) which makes use of the fluctuation of intracellular Ca⁺⁺ and CAMP concentrations, and more preferred are the methods described in Example 5 and Example 7.

### a) A screening method which uses a ligand binding assay method

The substances (compounds, peptides and antibodies) which bind to the LTB₄ receptor of the invention can be screened by a ligand binding assay method. A cell or cell membrane expressing the receptor or a purified sample of the receptor is prepared. Buffer, ion, pH and the like assay conditions are optimized, and a cell membrane expressing the receptor or a purified sample of the receptor is incubated for a predetermined period of time in the optimized buffer together with a labeled ligand such as [³H]LTB₄ and an agent to be tested. After the reaction, this is filtered using a glass filter or the like and washed with an adequate amount of the buffer, and then the radioactivity remained on the filter is measured using a liquid scintillation counter or the like. By the use of the thus obtained binding inhibition of radioactive ligand as the index, compounds, peptides and antibodies having the agonist activity for the receptor and compounds, peptides and antibodies having antagonist activity for the receptor can be screened.

In this connection, the inventors have elaborately searched for combinations of host cells and expression vectors and found as a result that it is desirable for this screening system to use a membrane fraction prepared by the combination of HEK293-EBNA with pCEP4-JULF2 described in Example 8.

### b) A screening method which uses a GTPγS binding method

It is possible to screen substances (compounds, peptides and antibodies) which modify the activity of the LTB₄ receptor of the invention by a GTPγS binding method (Lazareno, S. and Birdsall, N.J.M. (1993) Br. J. Pharmacol., 109, 1120 - 1127). A cell membrane expressing the receptor is mixed with 400 pM of ³⁵5-labeled GTPγS in a solution consisting of 20 mM HEPES (pH 7.4), 100 mM NaCl, 10 mM MgCl₂ and 50 mM GDP. After incubation in the presence or absence of an agent to be tested, the mixture is filtered using a glass filter or the like, and the radioactivity of the bonded GTPγS is measured using a liquid scintillation counter or the like. Using increase in the specific GTPγS binding in the presence of the agent to be tested as the index, compounds, peptides and antibodies having agonist activity for the receptor can be screened. Also, using inhibition of the increase in GTPγS binding by LTB₄ in the presence of the agent to be tested as the index, compounds, peptides and antibodies having antagonist activity for the receptor can be screened.

### c) A screening method which uses fluctuation of intracellular Ca⁺⁺ and cAMP concentrations

It is possible to screen substances (compounds, peptides and antibodies) which modify the activity of the LTB₄ receptor of the invention by making use of the fluctuation of intracellular Ca⁺⁺ or cAMP concentration in cells expressing the LTB₄ receptor. The Ca⁺⁺ concentration can be measured using fura2, fluo3 and the like, and the cAMP concentration can be measured using a commercially available cAMP assay kit (e.g., mfd. by Amersham). Also, it is possible to measure Ca⁺⁺ and CAMP concentrations indirectly, by detecting transcription activity of a gene whose transcription quantity is controlled depending on the Ca⁺⁺ and CAMP concentrations. An agent to be tested is allowed to react for a predetermined period of time with a cell expressing the receptor and a cell not expressing the receptor (control cell), and the Ca⁺⁺ and CAMP concentrations are measured directly or indirectly. Compounds, peptides and antibodies having agonist activity for the receptor can be screened making use, as the index, of the increased Ca⁺⁺ and/or decreased cAMP concentration specific for the receptor-expressed cell in comparison with the control cell. Also, compounds, peptides and antibodies having antagonist activity for the receptor can be screened making use, as the index, of the action of LTB₄ to inhibit increase of Ca⁺⁺ and/or decrease of cAMP concentration in the presence of an agent to be tested. A substance preferably showing EC₅₀ = 100 µM or less, more preferably a substance showing EC₅₀ = 10 µM or less, under the conditions described in Example 5 can be selected as a substance having the agonist activity. Also, a substance preferably showing IC₅₀ = 10 µM or less, more preferably a substance showing IC₅₀ = 1 µM or less, when an agent to be tested is added to the assay conditions described in Example 5, namely under the conditions of Example 7, can be selected as a substance having the antagonist activity.

In this connection, regarding the cAMP measuring test, the detection sensitivity "Signal/Noise ratio" (S/N ratio) decreases as the expressed amount of LTB₄ receptor increases. Accordingly, it is desirable to use a cell from which the highest S/N ratio can be obtained, namely a stably expressing cell strain having not so high receptor expression quantity, as the receptor expression cell to be used in this screening.

### 4) Method for preparing antibodies which react with the LTB₄ receptor of the invention

The antibodies of the invention can be produced by the following methods.

Antibodies which react with the LTB₄ receptor of the invention, such as a polyclonal antibody and a monoclonal antibody, can be obtained by directly administering the receptor or a fragment of the receptor to various animals. They can also be obtained by the DNA vaccine method (Raz, E. *et al*. (1994) *Proc. Natl. Acad. Sci. USA*, 91, 9519 - 9523; Donnelly, J.J. *et al.* (1996) *J. Infect. Dis*., 173, 314 - 320) using a plasmid into which a nucleic acid coding for the LTB₄ receptor of the invention is introduced.

A polyclonal antibody is produced from sera or eggs of rabbit, rat, goat, domestic fowl or the like animal which is immunized and sensitized by emulsifying the receptor or a fragment thereof in complete Freund's adjuvant or the like appropriate adjuvant and administering the emulsion by intraperitoneal, subcutaneous, intravenous or the like injection. The polyclonal antibody produced from sera or eggs in this manner can be separated and purified by conventional protein isolation purification methods. Examples of such methods include centrifugation, dialysis, salting out with ammonium sulfate and DEAE-cellulose, hydroxyapatite, protein A agarose or the like chromatography. Preferably, it can be produced using a peptide having the amino acid sequence described in SEQ ID NO:7 as the antigen under the conditions described in Example 3.

A monoclonal antibody can be produced easily by those skilled in the art by the cell fusion method of Kohler and Milstein (Kohler, G. and Milstein, C. (1975) *Nature*, 256, 495 - 497).

Immunization is carried out by inoculating an emulsion prepared by emulsifying the LTB₄ receptor of the invention or a fragment thereof in complete Freund's adjuvant or the like appropriate adjuvant, into the abdominal cavity, under the skin or into a vein of a mouse several times at intervals of several weeks. After the final immunization, spleen cells are taken out and fused with a myeloma cell to prepare a hybridoma.

As the myeloma cell for obtaining a hybridoma, a myeloma cell having hypoxanthine-guanine phosphoribosyltransferase deficiency, thymidine kinase deficiency or the like marker, such as a mouse myeloma cell strain P3X63Ag8.U1, is used. Also, polyethylene glycol is used as the fusing agent. In addition, as the medium to be used in the hybridoma preparation, Eagle's minimum essential medium, Dulbecco's modified minimum essential medium, RPMI-1640 or the like generally and frequently used medium is used by optionally supplementing it with 10 to 30% of fetal bovine serum. The fused strains are selected by the HAT selection method. Screening of hybridoma is carried out by ELISA method, immunological tissue staining method or the like known method or the aforementioned screening method using culture supernatants, and a clone of hybridoma secreting the antibody of interest is selected. Also, monoclonal nature of the hybridoma is guaranteed by repeating its subcloning by the limiting dilution method. A purification-possible amount of the antibody is produced by culturing the thus obtained hybridoma in a medium for 2 to 4 days or in the abdominal cavity of a pristane-pretreated BALB/c mouse for 10 to 20 days.

The monoclonal antibody produced in this manner can be separated and purified from the culture supernatant or ascitic fluid by conventional protein isolation purification methods. Examples of such methods include centrifugation, dialysis, salting out with ammonium sulfate and DEAE-cellulose, hydroxyapatite, protein A agarose or the like chromatography. In addition, a monoclonal antibody or an antibody fragment containing a part thereof can also be produced by integrating the entire or a partial portion of a nucleic acid coding for the antibody into an expression vector and introducing into *E. coli*, yeast or animal cells.

By digesting the thus separated and purified antibody with pepsin, papain or the like proteolytic enzyme in the usual way and subsequently carrying out separation purification by the conventional protein isolation purification methods, an active antibody fragment containing a part of the antibody, such as F(ab')₂, Fab, Fab', or Fv, can be obtained.

In addition, it is possible to obtain an antibody which reacts with the LTB₄ receptor of the invention as single chain Fv or Fab by the methods of Clackson and Zebedee (Clackson, T. *et al.* (1991) *Nature,* 352, 624 - 628; Zebedee, S. *et al.* (1992) *Proc. Natl. Acad. Sci. USA*, 89, 3175 - 3179). Also, it is possible to obtain a human antibody by immunizing a transgenic mouse in which a mouse antibody DNA is replaced by a human antibody DNA (Lonberg, N. *et al*. (1994) *Nature*, 368, 856 - 859).

### 5) Pharmaceuticals of the invention

Pharmaceuticals which use, as the active ingredients, substances (compounds; peptides and antibodies) that modify the activity of the LTB₄ receptor of the invention and can be selected by the screening method described in 3) are included in the invention. The pharmaceutical composition of the invention is preferably a pharmaceutical composition for inflammatory diseases, which uses a substance having antagonist activity for the receptor as the active ingredient.

Examples of the active ingredient include, e.g. as substances having antagonist activity for the LTB₄ receptor of the invention, 4-hexyloxy-N-hydroxybenzenecarboximidoamide and the like selected in Example 7. The invention includes not only a pharmaceutical which uses 4-hexyloxy-N-hydroxybenzenecarboximidoamide as the active ingredient but also all pharmaceuticals which use a substance capable of modifying the activity of the receptor as the active ingredient, but it is desirable to use a substance having antagonist activity for the receptor as the active ingredient.

Pharmaceutical preparations which use, as the active ingredient, a substance (a compound, a peptide, an antibody or an antibody fragment) capable of modifying the activity of the LTB₄ receptor of the invention (preferably having the antagonist activity) are prepared using carriers, fillers and other additives generally used for their preparation, in response to the type of the active ingredient.

Examples of the administration include oral administration by tablets, pills, capsules, granules, fine subtilaes, powders, oral solutions and the like or parenteral administration by intravenous, intramuscular or the like injections, suppositories, percutaneous preparations, transmucosal preparations and the like. Particularly in the case of peptides which are digested in the stomach, intravenous injection or the like parenteral administration is desirable.

In the solid composition for oral administration according to the invention, one or more active substances are mixed with at least one inert diluent such as lactose, mannitol, glucose, microcrystalline cellulose, hydroxypropylcellulose, starch, polyvinyl pyrrolidone, aluminum magnesium silicate and the like. In the usual way, the composition may contain other additives than the inert diluent, such as a lubricant, a disintegrating agent, a stabilizing agent, a solubilizing or solubilization assisting agent and the like. If necessary, tablets or pills may be coated with a sugar coat or a film of a gastric or enteric substance.

The liquid composition for oral administration includes emulsions, solutions, suspensions, syrups and elixirs and contains a generally used inert diluent such as purified water or ethanol. In addition to the inert diluent, this composition may also contain other additives such as moistening agent, suspending agents, sweeteners, aromatics and antiseptics.

The injections for parenteral administration includes aseptic aqueous or non-aqueous solutions, suspensions and emulsions. Examples of the diluent for use in the aqueous solutions and suspensions include distilled water for injection, physiological saline and the like. Examples of the diluent for use in the non-aqueous solutions and suspensions include propylene glycol, polyethylene glycol, olive oil or the like plant oil, ethanol or the like alcohol, polysorbate 80 and the like. Such a composition may further contain a moistening agent, an emulsifying agent, a dispersing agent, a stabilizing agent, a solubilizing or solubilization assisting agent, an antiseptic and the like. These compositions are sterilized by filtration through a bacteria retaining filter, blending of a germicide or irradiation. Alternatively, they may be used by firstly producing sterile solid compositions and dissolving them in sterile water or a sterile solvent for injection use prior to their use.

The clinical dose is optionally decided by taking into consideration strength of the activity of each active ingredient selected by the aforementioned screening method, symptoms and age, sex and the like of each patient to be treated.

For example, in the case of oral administration, the dose is generally from about 0.1 to 100 mg, preferably from 0.1 to 50 mg, per day per adult (as 60 kg in body weight). In the case of parenteral administration, it is from 0.01 to 50 mg, preferably from 0.01 to 10 mg, per day in the form of injections.

A method for using a DNA coding for the LTB₄ receptor as a diagnostic agent is also included in the invention. Detection of a mutation type LTB₄ receptor gene related to functional disorders is used in the diagnosis of diseases induced by too small expression, excess expression or changed expression of the LTB₄ receptor, or of morbidity thereof. Accordingly, the invention relates to a DNA which specifically hybridizes with the DNA represented by the nucleotide sequence described in SEQ ID NO:1 and has a chain length of at least 15 nucleotides. The term "specifically hybridizes" with the DNA of the invention means that it hybridizes with the DNA of the invention but does not hybridize with other DNA under general hybridization conditions, preferably under "stringent conditions". It is possible to use such a DNA as a probe for detecting and isolating the DNA of the invention and as a primer for amplifying the DNA of the invention. When used as a probe, a DNA which has at least a part of or the entire sequence (or a complimentary sequence thereof) of the DNA of the invention and has a chain length of at least 15 nucleotides is used. The nucleotide sequence desirable as the probe is from the 22nd position to the 615th position of SEQ ID NO:1 used in Example 4-1. The "under stringent conditions" are preferably the conditions described in Example 4-1. Also, when used as a primer, it has a chain length of generally from 15 to 100 nucleotides, preferably from 15 to 40 nucleotides. Nucleotide sequences desirable as the primer are shown in SEQ ID NO:3 (forward primer) and SEQ ID NO:4 (reverse primer) and SEQ ID NO:8 (forward primer) and SEQ ID NO: 9 (reverse primer).

The DNA for diagnosis use can be obtained from cells of persons to be diagnosed, such as blood, urine, saliva and a biopsy or partial biopsy material of tissue. Deletion and insertion mutations can be detected by changes in the size of amplified product when compared with normal genotype. Point mutation can be identified by hybridizing amplified DNA with labeled LTB₄ receptor nucleotide. Completely matched sequence and mismatched double strand can be distinguished by RNase digestion or difference in the melting temperature. Also, difference in the DNA sequence can be detected by changes in the mobility of DNA fragments by electrophoresis with a gel using or without using a denaturing agent, or by directly determining the DNA sequence (Myers, R.M. *et al.* (1985) *Science,* 230, 1242 - 1246). Changes in the sequence at a specific position can be confirmed by nuclease protection assay (e.g., RNase and S1 protection) or by a chemical cleavage method (Cotton *et al.* (1985) *Proc. Natl. Acad. Sci. USA,* 85, 4397 - 4401). Also, an array of oligonucleotide probes containing a nucleic acid coding for the LTB₄ receptor of the invention or a fragment thereof can be constructed. This array technique is well known and used for the analysis of gene expression, genetic linkage and genetic variability (Chee, M. *et al.* (1996) *Science,* 274, 610 - 613). In addition, this is used in the diagnosis of diseases induced by too small expression, excess expression or changed expression of the receptor, or of morbidity thereof, by a method which measures abnormal decrease or increase of the receptor level from samples obtained from subjects. The decrease or increase of expression can be measured at the RNA level by any one of polynucleotide determination methods well known to those skilled in the art, such as PCR, RT-PCR, RNase protection, northern blot technique and other hybridization methods. Also, decrease or increase of the receptor level from samples obtained from subjects can be measured by an assay method well known to those skilled in the art. Examples of such an assay method include radioimmunoassay, competitive binding assay, western blotting, ELISA and the like.

### Brief Description of the Drawings

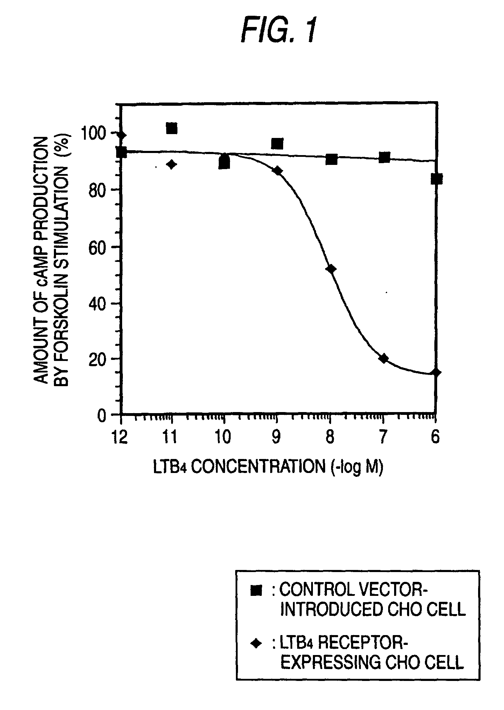

Fig. 1 is a graph showing a result of intracellular cAMP inhibition action of LTB₄ for LTB₄ receptor. In the drawing, the ordinate shows produced amount of CAMP when 1 µM forskolin alone stimulation is defined as 100% and unstimulation as 0%, and the abscissa shows LTB₄ concentration (-log M) in the reaction solution.

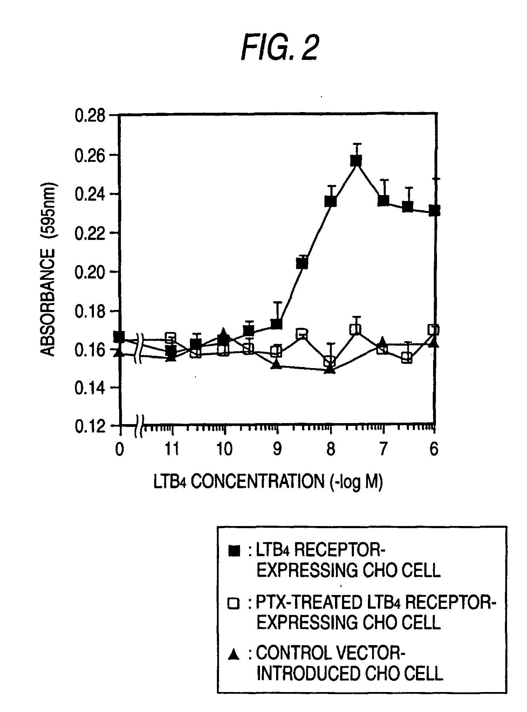

Fig. 2 is a graph showing a result of a cell migration test of LTB₄ receptor-stably expressing CHO cell by LTB₄. In the drawing, the ordinate shows absorbance (595 nm), and the abscissa shows LTB₄ concentration (-log M) in the lower layer solution.

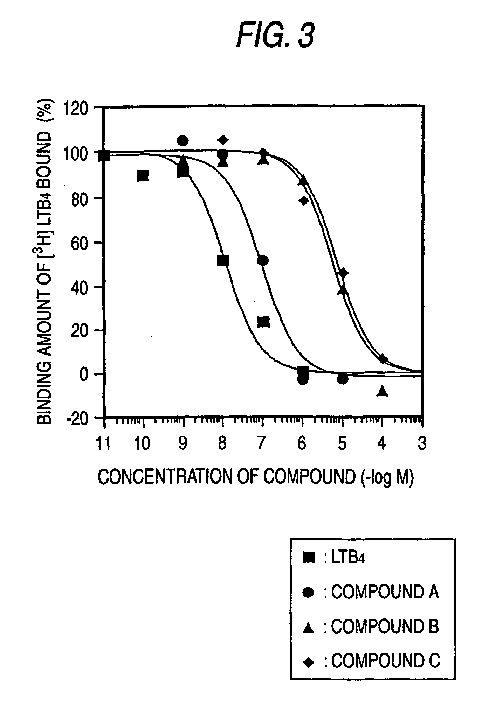

Fig. 3 is a graph showing a result of dose-dependent inhibition of the binding of [³H]LTB₄ to LTB₄ receptor by compound A, compound B and compound C. In the drawing, the ordinate shows binding amount of [³H]LTB₄ when total binding amount is defined as 100% and non-specific binding amount as 0%, and the abscissa shows concentration (-log M) of LTB₄, compound A, compound B and compound C in the reaction solution.

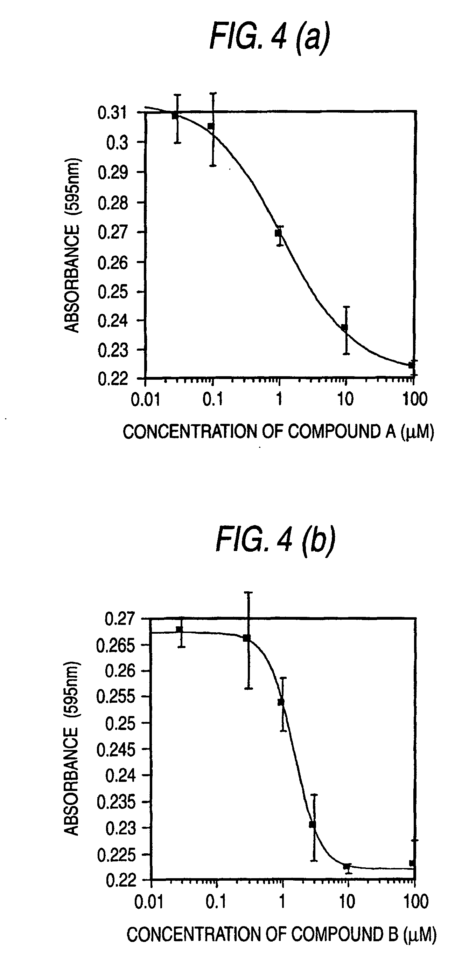

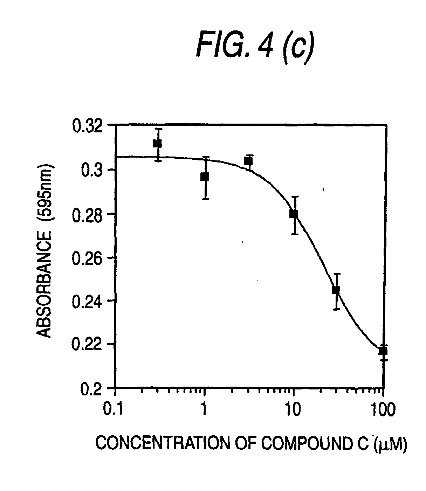

Fig. 4 is a graph showing a result of dose-dependent inhibition of compound A (Fig. 4 (a)), compound B (Fig. 4 (b)) and compound C (Fig. 4 (c)) for cell migration of LTB₄ receptor-stably expressing CHO cell toward LTB₄. In the drawing, the ordinate shows absorbance (595 nm), and the abscissa shows compound concentration (µM) in the upper layer solution.

### Best Mode for Carrying Out the Invention

The following describes the invention in detail by examples, but the invention is not restricted by these examples. In this connection, unless otherwise noted, they can be carried out in accordance with known methods (Maniatis, T. *et al.* (1982): "Molecular Cloning - A Laboratory Manual" Cold Spring Harbor Laboratory, NY). (Example 1) Isolation of DNA coding for novel receptor JULF2

Complete length cDNA coding for a novel receptor of the invention (to be referred to as JULF2 hereinafter) was obtained by PCR using a human genomic DNA (mfd. by Clontech) as the template. The oligonucleotide represented by SEQ ID NO:3 was used as a forward primer, and the oligonucleotide represented by SEQ ID NO:4 as a reverse primer (*Xba*I site is added to each of the respective 5'-ends). The PCR was carried out using Pfu DNA polymerase (mfd. by Stratagene) by repeating a cycle of 98°C (20 seconds)/58°C (30 seconds)/74°C (3 minutes) 34 times in the presence of 5% DMSO. As a result, a DNA fragment of about 1.0 kbp was amplified. This fragment was digested with *Xba*I and then cloned using pEF-BOS-dhfr plasmid (Mizushima, S. and Nagata, S. (1990) Nucleic Acids Res., 18, 5322). Nucleotide sequence of the thus obtained clone was analyzed by the dideoxy chain termination method using ABI377 DNA Sequencer (mfd. by Applied Biosystems). The thus revealed sequence is shown in SEQ ID NO:1.

The nucleotide sequence represented by SEQ ID NO:1 has an open reading frame (ORF) 1,077 bases. An amino acid sequence (358 amino acids) deduced from the ORF is shown in SEQ ID NO:2. Since the deduced amino acid sequence has hydrophobic regions considered to be 7 transmembrane domains which are a characteristic of G protein-coupled receptor, it was found that this JULF2 DNA encodes a G protein-coupled receptor.

### (Example 2) Confirmation of expression of novel receptor JULF2

As an expression vector for expressing JULF2, a plasmid pEF-BOS-dhfr-FL in which the oligonucleotide represented by SEQ ID NO:5 was inserted into the *Xba*I site of the plasmid pEF-BOS-dhfr described in Example 1 was used. By inserting a gene of interest into the *Xba*I site of this plasmid and expressing it, a FLAG epitope represented by the amino acid sequence described in SEQ ID NO:6 is fused as a marker to the N-terminus of the protein of interest. A plasmid constructed by inserting the JULF2 gene into pEF-BOS-dhfr-FL was named pEF-BOS-dhfr-FL-JULF2.

see293-EBNA (mfd. by Invitrogen) was inoculated into a 15 cm Petri dish at a density of 5 x 10⁶ cells and cultured for 1 day, and then 20 µg of pEF-BOS-dhfr-FL-JULF2 or pEF-BOS-dhfr-FL was subjected to gene transfer using Lipofectamine-2000 (mfd. by Gibco BRL). After the gene transfer, the cells were cultured for 1 day, recovered and washed, and then a 2 x 10⁵ portion of the cells were allowed to react with 1,000 times-diluted mouse anti-FLAG monoclonal antibody M2 (mfd. by Sigma) or control normal mouse IgG (mfd. by Zymed) as the primary antibody and then with FITC-labeled goat anti-mouse IgG polyclonal antibody (mfd. by BIOSOURSE) as the secondary antibody. When the cells stained in this manner were measured by a flow cytometer (EPICS® XL-MCL; mfd. by Coulter), it was confirmed that the FLAG epitope-fused JULF2 was expressed on the HEK293-EBNA cell membrane surface in the case of the cells into which pEF-BOS-dhfr-FL-JULF2 had been introduced.

Next, each of the HEK293-EBNA cell in which expression of the FLAG epitope-fused JULF2 on the cell membrane surface was confirmed (to be referred to as FLAG-JULF2 expressing HEK293-EBNA cell hereinafter) and the HEK293-EBNA cell into which pEF-BOS-dhfr-FL was introduced (to be referred to as FLAG expressing HEK293-EBNA cell hereinafter) was suspended in 20 mM Tris-HCl (pH 7.4)/150 mM NaCl/Complete™ (mfd. by Boehringer Mannheim) and homogenized using Polytron. SDS, digitonin and sodium cholate were added to the homogenate to final concentrations of 4%, 0.1% and 0.2% and incubated at 4°C for 2 hours, and then 20% v/v of SDSOut (mfd. by PIERCE) was added thereto and incubated at 4°C for 30 minutes for the purpose of removing free SDS. Using a supernatant treated at 10,000 x *g* for 10 minutes as a solubilized sample, immunoprecipitation of the FLAG epitope-fused JULF2 was carried out using M2-agarose (mfd. by Sigma). The immune precipitate was washed, treated at 100°C for 5 minutes in a sample buffer [0.25 M Tris-HCl (pH 6.8), 10% glycerol, 2% SDS, 0.1% Bromophenol Blue], subjected to electrophoresis using SDS/10% - 20% acrylamide gel (mfd. by Daiichi Pure Chemicals) and then transferred on a PVDF membrane using a blotting apparatus. The PVDF membrane after the transfer was subjected to blocking and then allowed to react with mouse anti-FLAG monoclonal antibody M2 (mfd. by Sigma) and horseradish peroxidase-labeled rabbit anti-mouse IgG polyclonal antibody (mfd. by Zymed) in that order. After the reaction, expression of FLAG epitope-fused JULF2 was confirmed using ECL Western Blotting Detection System (mfd. by Amersham Pharmacia).

The FLAG epitope-fused JULF2 was detected as a band of 37 to 45 kDa which, among proteins capable of reacting with the anti-FLAG monoclonal antibody M2, does not exist in the FLAG expressing HEK293-EBNA cell but exists in the FLAG-JULF2 expressing HEK293-EBNA cell. Estimated molecular weight of JULF2 was 38 kDa, and its band was found as almost expected molecular weight.

### (Example 3) Preparation of antibody for human JULF2

A peptide represented by SEQ ID NO:7 (JULF2-C22) prepared by adding cysteine to a partial amino acid sequence (from the 338th position to the 358th position of SEQ ID NO:2) of JULF2 was linked to KLH using Imject Maleimide Activated mcKLH Kit (mfd. by PIERCE) and used as an immunization antigen for the preparation of antibody for human JULF2. This was emulsified by mixing with TiterMax Gold (mfd. by CytRX) and administered under the dorsal skin of Japanese white rabbit (6 weeks of age). The dose was firstly 1 mg, and then administered twice in 0.5 mg portions at intervals of 2 weeks. After the final immunization, blood was collected to prepare anti-JULF2-C22 polyclonal antibody.

Western blotting was carried out using the anti-FLAG antibody M2 immune precipitates of FLAG-JULF2 expressing HEK293-EBNA cell and FLAG expressing HEK293-EBNA cell prepared in Example 2 and rabbit anti-JULF2-C22 polyclonal antibody. Illustratively, each immune precipitate was subjected to electrophoresis using SDS/10% - 20% acrylamide gel (mfd. by Daiichi Pure Chemicals) and then transferred on a PVDF membrane using a blotting apparatus. The PVDF membrane after the transfer was subjected to blocking and then allowed to react with 1,000 times-diluted rabbit anti-JULF2-C22 polyclonal antibody and 2,000 times-diluted horseradish peroxidase-labeled goat anti-rabbit IgG polyclonal antibody (mfd. by MBL) in that order. After the reaction, color development was carried out using the ECL Western Blotting Detection System. The band reacting with the anti-JULF2-C22 polyclonal antibody was detected as a band of 37 to 45 kDa at the same position of the anti-FLAG antibody M2 of Example 2 only with the FLAG-JULF2 expressing HEK293-EBNA cell.

It was shown by the above result that the anti-JULF2-C22 polyclonal antibody is an antibody which recognizes JULF2. The use of this antibody rendered possible detection of natural JULF2 by western blotting, immunological tissue staining and the like methods.

### (Example 4-1) Expression distribution of human JULF2 DNA in tissues

Expression distribution of JULF2 DNA was analyzed by northern blot hybridization method. As the probe of human JULF2, a cDNA fragment (from the 22nd position to the 615th position of SEQ ID NO:1) was used. Hybridization of a membrane on which poly(A)⁺ RNA (2 µg) derived from each human organ was blotted (mfd. by Clontech) with the probe was carried out at 42°C (18 hours) in a solution containing 50% formamide, 5 x SSPE. 10 x Denhardt's solution, 2% SDS and 100 µg/ml denatured salmon sperm DNA. The membrane was finally washed twice (65°C , 30 minutes) with a solution containing 0.2 x SSC and 0.1% SDS.

When northern analysis was carried out on each of human tissues (the heart, brain, placenta, lung, liver, skeletal muscle, kidney, pancreas, spleen, thymus, prostate, testis, ovary, small intestine, large intestine, peripheral blood leukocyte, stomach, thyroid gland, spinal cord, lymph node, trachea, adrenal gland and bone marrow), mRNA of about 2.5 kb was strongly detected in the spleen, peripheral blood leukocyte, adrenal gland, bone marrow, trachea, thyroid gland, ovary and lymph node, and the signal was slightly detected in the pancreas, heart and spinal cord. Also, mBNA molecules of 3.5 kb, 5 kb and 9.5 kb were expressed in peripheral blood leukocyte, particularly, the mRNA of 5 kb was strongly detected in the spleen, bone marrow and adrenal gland. In addition, a mRNA of 6.5 kb was specifically detected only in the trachea. It was found that at least five different sizes of mRNA for the G protein-coupled receptor human JULF2 DNA are expressed in a broad range of human tissues. Based on the above, while BLT as a known LTB₄ receptor was expressed only in peripheral blood leukocyte, gene expression of the JULF2 of the invention was confirmed in a broad range including the spleen, peripheral blood leukocyte and bone marrow, so that a possibility was suggested that it is taking an important role in a broad range of physiological actions originated from LTB₄. In addition, since gene expression of the BLT as a known LTB₄ receptor was hardly found in the spleen but gene expression of the JULF2 of the invention was found in the spleen, a possibility was suggested that JULF2 is generally expressed in blood cells including mononuclear cells (lymphocyte, monocyte).

### (Example 4-2) Expression distribution of novel human JULF2 DNA in blood cells

Heparin blood samples were collected from healthy volunteers, mixed with 1/3 volume of 6% dextran/physiological saline and then allowed to stand at room temperature for 1 hour. The supernatant was collected and centrifuged at 150 x g for 5 minutes, and the sediment was suspended in Hunk's balanced salt solution (HBSS). This was overlaid on the same volume of Ficoll (Pharmacia) and centrifuged at 400 x g for 30 minutes. The intermediate layer was collected as a mononuclear cell fraction, and the sediment as polynuclear leukocytes. The polynuclear leukocytes were mixed with CD16 microbeads (mfd. by Daiichi Pure Chemicals) and separated into a neutrophil fraction and eosinophil fraction by a porcelain stand. Each of the mononuclear cell fraction, neutrophil fraction and eosinophil fraction was washed with physiological saline, and then total RNA was purified using Isogen (mfd. by Nippon Gene). A 5 µg portion of the total RNA derived from each fraction was allowed to undergo the reaction at 37°C for 15 minutes using a DNase (mfd. by Nippon Gene). The total BNA treated with DNase was converted into cDNA using Superscript First Strand System (for RT-PCR) (mfd. by GIBCO).

Expression distribution of JULF2 was measured using the blood cell fraction cDNA as the template and using the oligonucleotide represented by SEQ ID NO:8 and oligonucleotide represented by SEQ ID NO:9 as the primer . set. The PCR was carried out using Pfu DNA polymerase (mfd. by Stratagene) by repeating a cycle of 98°C (20 seconds)/60°C (30 seconds)/74°C (2 minutes) 35 times in the presence of 5% DMSO. Also, as an internal standard, PCR was carried out under the same conditions using the aforementioned cDNA of each human organ as the template and using Human G3PDH Control Amplimer Set (mfd. by Clontech). Also, in order to verify contamination of genomic DNA, total RNA (RT(-)) which was not converted into cDNA was used as a control. The reaction products were analyzed by 1% agarose gel electrophoresis. About 400 bp amplification product of JULF2 was detected in each of the blood cell fractions of both of the healthy persons A and B, particularly in the mononuclear cell.

Since JULF2 was significantly detected in mononuclear cell while the BLT as a known LTB₄ receptor is considered to be expressed frequently in neutrophil, it was suggested that JULF2 is mostly concerned in chronic inflammatory diseases among inflammatory diseases induced by leukotriene B₄.

### (Example 5) Establishment of JULF2 stably expressing CHO cell strain and cAMP production inhibition test toward LTB₄

As an expression vector for expressing human JULF2, pEF-BOS-dhfr was used. The thus constructed plasmid was named pEF-BOS-dhfr-Jul22.

The CHO-dhfr(-) strain was inoculated at a density of 1 x 10⁶ cells into a 10 cm Petri dish containing αMEM (supplemented with nucleic acids) medium/10% fetal bovine serum and cultured for 1 day and then subjected to gene transfer of 8 µg of pEF-BOS-dhfr-Jul22 and pEF-BOS-dhfr (control vector) using FuGENE6 (mfd. by Boehringer Mannheim). The gene-transferred cells were recovered 24 hours thereafter, suspended in αMEM (nucleic acid-free) medium/100 nM methotrexate (mfd. by Wako Pure Chemical Industries)/10% fetal bovine serum and then serially diluted and inoculated again into the 10 cm Petri dish. Colonies formed after 2 weeks were separately isolated and used as JULF2 stably expressing CHO cells.

Each of the JULF2 stably expressing CHO cells and control vector-introduced CHO cells was inoculated into a 24 well plate at a density of 1 x 10⁵ cells. After 1 day of culturing, the cells were treated for 10 minutes with α MEM (nucleic acid-free) medium/1 mM 3-isobutyl-1-methylxanthine (mfd. by Sigma)/0.1% BSA, and then 1 µM forskolin (mfd. by Wako Pure Chemical Industries)/0 to 1 µM LTB₄ was added dropwise thereto. After 30 minutes at 37°C, the culture supernatant was discarded and the cells were lysed using a cAMP EIA System (BIOTRAK; mfd. by Amersham) cell lysis solution.

Measurement of the produced amount of cAMP in cells under each condition was carried out using the cAMP EIA System in accordance with the attached protocol. By defining the produced amount of cAMP by stimulation with 1 µM forskolin alone as 100%, a dose-dependent curve of the amount of cAMP in the presence of LTB₄ was prepared (Fig. 1). According to the dose-dependent curve, response of LTB₄ for JULF2 was EC₅₀ = 10 nM or less. On the other hand, changes in the produced amount of CAMP by the addition of LTB₄ were not found in the control vector-introduced CHO cells. In addition, since the produced amount of CAMP in the JULF2 stably expressing CHO cells by forskolin stimulation was inhibited by LTB₄, it was suggested that JULF2 is coupled with Gai among the intracellular trimer G proteins.

### (Example 6) Cell migration test of JULF2 stably expressing CHO cells toward LTB₄

A 8 µm pore polycarbonate frame filter (mfd. by Neuroprobe) was treated with 10 µg/ml fibronectin (Asahi Technoglass)/PBS at 4°C overnight. From 0 to 1 µM of LTB₄ was put into the lower layer of 96 blind well chamber (mfd. by Neuroprobe), the fibronectin-treated frame filter was set, and the JULF2 stably expressing CHO cells and control vector-introduced CHO cells were suspended in α MEM (nucleic acid-free) medium/0.1% BSA and then inoculated into the upper layer of the chamber at a density of 2 x 10⁵ cells. After 4 hours of culturing at 37°C in a CO₂ incubator, the frame filter was fixed with methanol and stained with Diff-Quik Staining Kit (International Reagents Corporation). The upper surface of this filter (the side on which cells were set) was wiped and air-dried and then absorbance at 595 nm was measured using a plate reader (Molecular Devices). Also, in order to examine pertussis toxin (PTX) sensitivity of the JULF2 stably expressing CHO cells, JULF2 stably expressing CHO cells treated with 50 ng/ml PTX for 20 hours were subjected to a cell migration test (Fig. 2). It was observed that the JULF2 stably expressing CHO cells migrate into the lower layer of the filter toward LTB₄. The cell migration showed a bell type chemotaxis in which the migration activity becomes maximum for 30 nM concentration of LTB₄ and in which the migration activity is inhibited at further high concentration. Since this reaction was completely inhibited by PTX pretreatment, it was strongly suggested that a Gαi-like G protein is concerned in the JULF2-mediated cell migration.

### (Example 7) Screening of compounds which inhibit CAMP inhibition action by LTB₄ using JULF2 stably expressing CHO cell

Using the JULF2 stably expressing CHO cell prepared in Example 5, screening of candidate compounds which inhibit cAMP inhibition action by LTB₄ was carried out. Illustratively, the JULF2 stably expressing CHO cell was inoculated into a 96 well plate at a density of 1 x 10⁴ cells and, after 1 day of culturing, treated for 10 minutes with a MEM (nucleic acid-free) medium/1 mM 3-isobutyl-1-methylxanthine (mfd. by Sigma)/0.1% BSA. After the treatment, a mixture of 1 µM forskolin (mfd. by Wako Pure Chemical Industries)/100 nM LTB₄ with 4.2 µM of each candidate compound was added dropwise thereto and incubated at 37°C for 30 minutes. After discarding the culture supernatant, the cells were lysed using 100 µl 0.2% Triton X-100/PBS, and the amount of CAMP in 5 µl of cell lysate was measured using Cyclic AMP kit (mfd. by CIS Bio International) in accordance with the attached protocol. Under conditions in which the produced amount of CAMP by stimulation with 1 µM forskolin alone was defined as 100%, and the amount of CAMP in the presence of 100 nM LTB₄ as 0%, compounds having an IC₅₀ value of 10 µM or less, such as 4-octyloxybenzenecarboximidoamide hydrochloride (to be referred to as compound A hereinafter), 4-hexyloxy-N-hydroxybenzenecarboximidoamide (to be referred to as compound B hereinafter), compound C (FAB-MS (M + H)⁺ 337) and the like, were obtained. The compound A, compound B and compound C are commercially available reagents, and compound A is on the market as a code number 0199-0032 of ChemDiv, Inc, and compound B as a code number TPB-488 of InterBioScreen. It was found that these compounds are antagonists of JULF2, because they inhibit CAMP inhibition action of JULF2 stably expressing CHO cell in a dose-dependent manner. Their IC₅₀ values are 0.4 µM in compound A, 6.7 µM in compound B and 12 µM in compound C. In this connection, the property of the compound A to show antiinflammatory effect is also described in *Biochemical Pharmacology*, 51, 737 - 742, 1996.

### (Example 8) Screening of compounds which inhibit binding of JULF2 and LTB₄ using JULF2 expressing cell membrane fraction

The DNA fragment obtained in Example 1 coding for JULF2 was digested with *Xba*I and then cloned into pCEP4 (mfd. by Invitrogen) to be used as an expression vector (pCEP4-JULF2) for expressing JULF2. HEK293-EBNA strain was inoculated into a 15 cm Petri dish at a density of 5 x 10⁶ cells and cultured for 1 day, and then 20 µg of pCEP4-JULF2 was subjected to gene transfer using Lipofectamine-2000 (mfd. by Gibco BRL). The gene-transferred cells were recovered 24 hours thereafter, washed, suspended in 20 mM Tris-HCl (pH 7.4)/5 mM EDTA and then homogenized using Polytron. After ultracentrifugation, the sediment was suspended in 20 mM Tris-HCl (pH 7.4) and used as a membrane fraction.

Using the JULF2 expressing cell membrane fraction, screening of candidate compounds was carried out making use of the activity to inhibit binding of LTB₄ as an index. Illustratively, a solution composed of 50 mM Tris-HCl(pH 7.4), 10 mM MgCl₂, 10 mM NaCl and 0.05% BSA, containing 100 µg of the JULF2 expressing cell membrane fraction, was mixed with 5 µM of a candidate compound and 0.5 nM in final concentration of [³H]LTB₄ and incubated at room temperature for 1 hour, and then the cell membrances were recovered on a glass filter using a cell harvester. A liquid scintillation was added to the glass filter and the radioactivity was measured using a liquid scintillation counter. Also, at the same time in the above test, radioactivity of a sample to which the candidate compound was not added and a sample to which 2 µM in final concentration of LTB₄ was measured as total binding amount and non-specific binding amount, respectively. Under such conditions, the compound A, compound B and compound C selected in Example 7 inhibited binding of the JULF2 expressing cell membrane fraction with LTB₄ in a dose-dependent manner. As shown in Fig. 3, their IC₅₀ values are 0.1 µM in compound A, 5.7 µM in compound B and 7.4 µM in compound C.

### (Example 9) Cell migration inhibition by JULF2 antagonist for cell migration of JULF2 stably expressing CHO cell toward LTB₄

In the cell migration system shown in Example 6, the cell migration activity was measured in the presence of the compound A, compound B or compound C selected in Example 7 in an amount of from 0.03 to 100 µM to the upper layer and adding 100 nM of LTB₄ to the lower layer. The results are shown in Fig. 4. It was confirmed that these compounds inhibit cell migration toward LTB₄ in a dose-dependent manner.

It is known that LTB₄ is a strong chemoattractant for neutrophil (Chen, X.S. *et al.* (1994) *Nature,* 372, pp. 179 - 182). Since JULF2 is expressed in mononuclear cell and also expressed in neutrophil as shown in Examples 4-1 and 4-2, it is considered that this receptor is concerned in the exacerbation of inflammatory diseases and allergic diseases, such as rheumatism, psoriasis, asthma and the like, via cell migration of these cells. Based on the above, it is considered that these JULF2 antagonists have anti-inflammation action by inhibiting cell migration.

### (Example 10) Cloning of rat JULF2 gene

Using a combination of the oligonucleotide represented by SEQ ID NO:10 with the oligonucleotide represented by SEQ ID NO:11 designed from the human JULF2 gene sequence information represented by SEQ ID NO:1, a DNA fragment was obtained by PCR. The PCR was carried out using rat genomic DNA (mfd. by Clontech) as the template by repeating a cycle of 98°C (20 seconds)/55°C (30 seconds)/74°C (3 minutes) 32 times in the presence of 5% DMSO. A DNA fragment of about 250 bp amplified as the result was analyzed using ABI377 DNA Sequencer. Next, based on the thus revealed rat JULF2 partial nucleotide sequence, the oligonucleotide represented by SEQ ID NO:12 and the oligonucleotide represented by SEQ ID NO:13 were prepared to carry out 5'-RACE and 3'-RACE using Rat Spleen Marathon cDNA Amplification Kit (mfd. by Clontech). The PCR was carried out in accordance with a usual method using LA Taq polymerase (mfd. by Takara Shuzo) to find that DNA fragments of about 2 kbp were amplified in both cases. Based on their sequence information, the oligonucleotide represented by SEQ ID NO:14 was used as a forward primer, and the oligonucleotide represented by SEQ ID NO:15 as a reverse primer, to carry out PCR (*Spe*I site is added to each of the respective 5'-ends). The PCR was carried out using rat spleen cDNA as the template and using Pfu DNA polymerase (mfd. by Stratagene) by repeating a cycle of 98°C (20 seconds)/70°C (30 seconds)/74°C (2 minutes) 12 times, a cycle of 98°C (20 seconds)/67°C (30 seconds)/74°C (2 minutes) 12 times and a cycle of 98°C (20 seconds)/64°C (30 seconds)/74°C (2 minutes) 16 times. As a result, a DNA fragment of about 1.0 kbp was amplified. This fragment was digested with *Spe*I and then cloned using pEF-BOS-dhfr plasmid (Mizushima, S. and Nagata, S. (1990) Nucleic Acids Res., 18, 5322). Nucleotide sequence of the thus obtained clone was analyzed by the dideoxy chain termination method using ABI377 DNA Sequencer (mfd. by Applied Biosystems). The thus revealed sequence is shown in SEQ ID NO:16. The nucleotide sequence represented by SEQ ID NO:16 has an ORF of 1,077 bases. An amino acid sequence (358 amino acids) deduced from the ORF is shown in SEQ ID NO:17. This amino acid sequence showed a homology of 92.2% with the amino acid sequence of human JULF2. Since homology of the BLT as a known LTB₄ receptor is 76.6% between human and rat and 75.5% between human and mouse, it was suggested that particularly JULF2 among LTB₄ receptors is an important receptor to be preserved during the steps of evolution.

### (Example 11) Establishment of rat JULF2 stably expressing CHO cell strain and intracellular cAMP production inhibition and cell migration tests toward LTB₄

The LTB₄ receptor activity of a protein encoded by the rat JULF2 DNA obtained in Example 10 was confirmed by the following tests. The pEF-BOS-dhfr plasmid was introduced with the rat JULF2 DNA obtained in Example 10 and named pEF-BOS-dhfr-rat JULF2.

In order to measure changes in the amount of intracellular CAMP, a rat JULF2 stably expressing CHO cell was obtained by the same method of Example 5. When changed amount of intracellular cAMP by LTB₄ in the presence of 1 µM forskolin (mfd. by Wako Pure Chemical Industries) was measured using the rat JULF2 stably expressing CHO cell under the same conditions of Example 5, LTB₄ dose-dependent cAMP production inhibition action was found. When analyzed by logistic regression, LTB₄ showed an EC₅₀ value of from 7.5 to 11.3 nM. In addition, as a result of the examination of the migration ability of rat JULF2 stably expressing CHO cell by the same method of Example 6, it showed a bell type chemotaxis in which the migration activity becomes maximum for 100 nM concentration of LTB₄ and in which the migration activity is inhibited at further high concentration.

Based on the above results, it was considered that response of rat JULF2 to LTB₄ is almost identical to the response of human JULF2.

### Industrial Applicability

The LTB₄ receptor provided by the invention is useful for the screening and evaluation of substances which modify the activity of the receptor as preventive and/or therapeutic agents for diseases induced by human leukotriene B₄, such as inflammatory diseases (bronchitis, psoriasis, ulcerative colitis, rheumatoid arthritis and edema), and substances useful as therapeutic agents for diseases in which this receptor is concerned can be selected by the screening method that uses the receptor provided by the invention. Also, the DNA coding for the receptor of the invention is useful not only for the production of the receptor but also for the diagnosis of diseases induced by mutation of or abnormal expressional changes in the receptor. The polyclonal antibody or monoclonal antibody for the receptor is useful for, e.g., the receptor agonists, diagnostic agents or means of separation and purification of polypeptides.

## Claims

1. A leukotriene B₄ receptor, which is represented by the amino acid sequence described in SEQ ID NO:2, or the amino acid sequence described in SEQ ID NO:2 in which one or more amino acid(s) residues are substituted, deleted and/or inserted at one or more position(s) and showing the same activity of the leukotriene B₄ receptor represented by the amino acid sequence described in SEQ ID NO:2.

2. The leukotriene B₄ receptor according to claim 1, which is represented by the amino acid sequence described in SEQ ID NO:2.

3. A leukotriene B₄ receptor, which is a protein encoded by a DNA that hybridizes with the DNA represented by the nucleotide sequence described in SEQ ID NO:1 or SEQ ID NO:16 under a stringent condition and showing the same activity of the leukotriene B₄ receptor represented by the amino acid sequence described in SEQ ID NO:2.

4. The leukotriene B₄ receptor according to claim 3, which is a protein represented by the amino acid sequence described in SEQ ID NO:2.

5. The leukotriene B₄ receptor according to claim 3, which is a protein represented by the amino acid sequence described in SEQ ID NO:17.

6. A nucleic acid which encodes the amino acid sequence of the leukotriene B₄ receptor described in claim 1 or 3.

7. A vector which contains the nucleic acid described in claim 6.

8. A host cell which contains the vector described in claim 7.

9. A method for producing the leukotriene B₄ receptor described in claim 1 or 3, **characterized in that** it uses the host cell described in claim 8.

10. An antibody which binds to the leukotriene B₄ receptor described in claim 1 or 3.

11. A method for screening a substance capable of modifying the activity of leukotriene B₄ receptor, **characterized in that** the leukotriene B₄ receptor described in claim 1 or 3 is allowed to contact with an agent to be tested.

12. The screening method according to claim 11, which comprises a step for contacting the leukotriene B₄ receptor described in claim 1 or 3 with an agent to be tested in the presence of a ligand and a step for measuring changes in the receptor activity.

13. The screening method according to claim 11, which comprises a step for contacting the leukotriene B₄ receptor described in claim 1 or 3 with an agent to be tested in the presence of a ligand and a step for measuring binding inhibition activity of the ligand for the receptor.

14. The screening method according to claim 11, wherein the substance capable of modifying the activity of the leukotriene B₄ receptor described in claim 1 or 3 is a substance for preventing and/or treating an inflammatory disease.

15. An antagonist of the leukotriene B₄ receptor described in claim 1 or 3, which is capable of being selected by the method described in claim 11.

16. A pharmaceutical composition for an inflammatory disease (excluding a pharmaceutical composition for an inflammatory disease in which 4-octyloxybenzenecarboximidoamide hydrochloride is the active ingredient), which contains as the active ingredient a substance that is capable of modifying the activity of the leukotriene B₄ receptor described in claim 1 or 3 and is capable of being selected by the method described in claim 11.

17. The pharmaceutical composition for an inflammatory disease according to claim 16 (excluding a pharmaceutical composition for an inflammatory disease in which 4-octyloxybenzenecarboximidoamide hydrochloride is . the active ingredient), wherein the substance capable of modifying the activity is a substance having antagonist activity.
